# EUROPEAN PATENT APPLICATION

(11) **EP 4 335 503 A2**
(43) Date of publication of application: **13.03.2024**
(21) Application number: 23210526.2
(22) Date of filing: 16.07.2020
(51) Int. Cl.: A61P 21/00

(54) **METHODS OF TREATING OR PREVENTING SPINAL MUSCULAR ATROPHY**

(30) Priority: 19.07.2019 US 201962876360 P
(62) Divisional of application: 20753556.8
(71) Applicant: Biogen MA Inc., Cambridge, MA 02142 (US)
(72) Inventor: LOVEDAY, Kenneth Swope, Cambridge, 02142 (US); BAI, Fengju Judy, Cambridge, 02142 (US); EAST, Lilly, Cambridge, 02142 (US); FARWELL, Wildon R., Cambridge, 02142 (US)
(74) Representative: Pohlman, Sandra M.

(57) **Abstract**

Featured are methods for the treatment or prevention of spinal muscular atrophy. Effective dosage regimens are specified. Biomarkers and kits are also provided.

## Description

### Cross-Reference to Related Application

This application claims priority to U.S. Provisional Appl. No. 62/876,360, filed July 19, 2019, the contents of which are incorporated by reference herein in their entirety.

### Technical Field

This disclosure relates generally to methods for treatment and prevention of spinal muscular atrophy and effective dosage regimens.

### Background

Spinal muscular atrophy (SMA) is a genetic, neurodegenerative disorder characterized by the loss of spinal motor neurons in the anterior horn of the spinal cord, resulting in atrophy of the voluntary muscles of the limbs and trunk. SMA is an autosomal recessive disease of early onset and with an incidence of 8.5 to 10.3 per 100,000 live births, it is the most common monogenetic cause of infant mortality and a major cause of childhood morbidity in the United States. The natural history of SMA includes 4 major recognized phenotypes that are dependent on age of onset and achieved motor abilities. Type I SMA (infantile-onset SMA) is the most severe form with onset at birth or within 6 months and typically results in death within 2 years. Children with type I SMA are unable to sit or walk. Later-onset SMA can be divided into Type II and Type III SMA. Type II SMA (presenting between 6 and 18 months of age) is the intermediate form and patients are able to sit, but cannot stand or walk. Patients with type III SMA typically develop SMA after 18 months of age and are able to sit and walk but may become severely and increasingly disabled. Adult-onset SMA or Type IV SMA patients have disease onset after 18 years of age and have normal life expectancy.

The molecular basis of SMA is the loss of both copies of survival motor neuron gene 1 (SMN1), which encodes a protein that is part of a multi-protein complex thought to be involved in snRNP biogenesis and recycling. A nearly identical gene, SMN2, exists in a duplicated region on chromosome 5q13 and modulates disease severity. Expression of the normal SMN1 gene results solely in expression of survival motor neuron (SMN) protein. Although SMN1 and SMN2 have the potential to code for the same protein, SMN2 contains a translationally silent mutation at position +6 of exon 7, which results in inefficient inclusion of exon 7 in SMN2 transcripts. Thus, the predominant form of SMN2 is a truncated version, lacking exon 7, which is unstable and inactive (Cartegni and Krainer, Nat. Genet., 30:377-384 (2002)). Expression of the SMN2 gene results in approximately 10-20% of the SMN protein and 80-90% of the unstable/non-functional SMNdelta7 protein. SMN protein plays a well-established role in assembly of the spliceosome and may also mediate mRNA trafficking in the axon and nerve terminus of neurons.

Humans have a variable copy number of the SMN2 gene (0 to 8 copies). The number of SMN2 copies and the resulting amount of full-length SMN protein expressed in patients with SMA correlate with SMA disease severity, and thus, SMN2 is a key modifier of disease phenotype.

Antisense technology is an effective means for modulating the expression of one or more specific gene products, including alternative splice products, and is uniquely useful in a number of therapeutic, diagnostic, and research applications. The principle behind antisense technology is that an antisense compound, which hybridizes to a target nucleic acid, modulates gene expression activities such as transcription, splicing or translation through one of a number of antisense mechanisms. The sequence specificity of antisense compounds makes them extremely attractive as tools for target validation and gene functionalization, as well as therapeutics to selectively modulate the expression of genes involved in disease. Certain antisense compounds complementary to SMN2 pre-mRNA are known in the art. See e.g., WO 2007/002390.

SPINRAZA^{®} is an antisense compound approved in the United States, Europe, and other countries and regions globally for the treatment of SMA in pediatric and adult patients at a recommended dosage of 12 mg. At the approved 12 mg dosage, SPINRAZA^{®} has a robust safety profile and has offered tremendous benefit to patients with SMA. In the context of the importance of early intervention, especially in patients with the most severe forms of SMA, Applicant has considered that more rapid achievement and maintenance of higher concentrations of SPINRAZA^{®} in patients with SMA would be beneficial.

### Summary

This application relates to methods of treating or preventing SMA. This disclosure features dosing regimens for antisense compounds described herein that rapidly achieve and maintain higher antisense compound concentrations in patients with SMA (relative to the approved SPINRAZA^{®} dosing regimen) and thus further enhance the efficacy of treatment.

In one aspect, the disclosure features a method for treating spinal muscular atrophy (SMA) in a human subject in need thereof. The method involves administering to the human subject at least two loading doses of an antisense compound having the following structure: 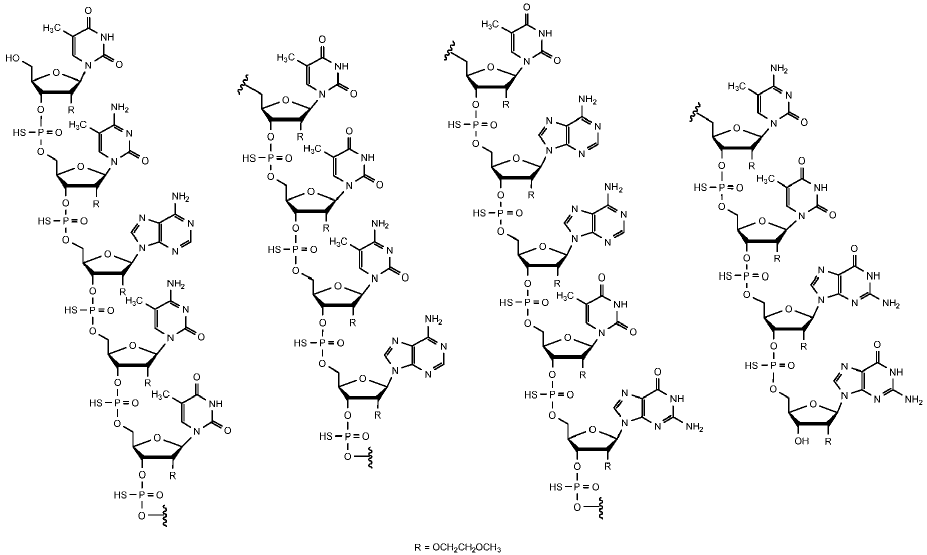 or a salt thereof. The administration is by intrathecal injection (e.g., intrathecal bolus injection) and comprises administering a first loading dose of the antisense compound or the salt thereof described herein that is equivalent to about 50 mg of the antisense compound; and a second loading dose of the antisense compound or the salt thereof described herein that is equivalent to about 50 mg of the antisense compound, administered about 10 to 20 days (e.g., about 10 to 18 days, about 12 to 16 days, or about 14 days) after administration of the first loading dose.

In another aspect, the disclosure features method for increasing levels of survival motor neuron-2 (SMN2) messenger ribonucleic acid (mRNA) containing exon 7 in a human subject having mutations in chromosome 5q that are associated with SMN protein deficiency.
The method involves administering to the human subject at least two loading doses of an antisense compound having the following structure: 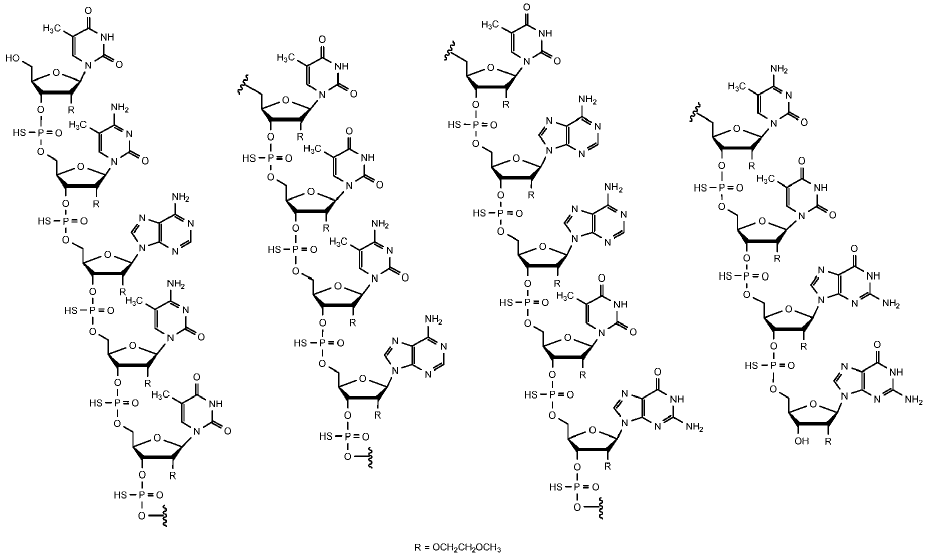 or a salt thereof. The administration is by intrathecal injection (e.g., intrathecal bolus injection) and comprises administering a first loading dose of the antisense compound or the salt thereof described herein that is equivalent to about 50 mg of the antisense compound; and a second loading dose of the antisense compound or the salt thereof described herein that is equivalent to about 50 mg of the antisense compound, administered about 10 to 20 days (e.g., about 10 to 18 days, about 12 to 16 days, or about 14 days) after administration of the first loading dose.

In some instances, the above methods further involve administering to the human subject by intrathecal injection (e.g., intrathecal bolus injection) at least one maintenance dose, wherein the at least one maintenance dose is equivalent to about 28 mg of the antisense compound.

In some instances, the above methods further comprise administering to the human subject by intrathecal injection (e.g., intrathecal bolus injection) maintenance doses of the antisense compound or the salt thereof described herein, beginning about 3 to 6 months (e.g., about 4 to 5 months; or about 4 months) after administration of the second loading dose and continuing once about 3 to 6 months (e.g., about 4 to 5 months; or about 4 months) thereafter, wherein each maintenance dose is equivalent to about 28 mg of the antisense compound.

In some instances, the second loading dose is administered about 14 days after administration of the first loading dose. In some instances, the second loading dose is administered 14 days after administration of the first loading dose.

In certain instances, the above methods further comprise administering to the human subject by intrathecal injection (e.g., intrathecal bolus injection) a third loading dose of the antisense compound or the salt thereof described herein that is equivalent to about 50 mg of the antisense compound, administered about 10 to 20 days (e.g., about 10 to 18 days, about 12 to 16 days, or about 14 days) after administration of the second loading dose; and one or more maintenance doses of the antisense compound or the salt thereof described herein, beginning about 3 to 6 months (e.g., about 4 to 5 months; or about 4 months) after administration of the third loading dose and continuing once about every 3 to 6 months (e.g., about 4 to 5 months; or about 4 months) thereafter, wherein each maintenance dose is equivalent to about 28 mg of the antisense compound. In some instances, the second loading dose is administered about 14 days after administration of the first loading dose and the third loading dose is administered about 14 days after administration of the second loading dose. In certain instances, the second loading dose is administered 14 days after administration of the first loading dose and the third loading dose is administered 14 days after administration of the second loading dose.

In some instances, the above methods further comprise administering to the human subject by intrathecal injection (e.g., intrathecal bolus injection) a third loading dose of the antisense compound or the salt thereof described herein that is equivalent to about 50 mg of the antisense compound, administered about 10 to 20 days (e.g., about 10 to 18 days, about 12 to 16 days, or about 14 days) after administration of the second loading dose; a fourth loading dose of the antisense compound or the salt thereof described herein that is equivalent to about 50 mg of the antisense compound, administered about 10 to 20 days (e.g., about 10 to 18 days, about 12 to 16 days, or about 14 days) after administration of the third loading dose; and one or more maintenance doses of the antisense compound or the salt thereof described herein, beginning about 3 to 6 months (e.g., about 4 to 5 months; or about 4 months) after administration of the fourth loading dose and continuing once about every 3 to 6 months (e.g., about 4 to 5 months; or about 4 months) thereafter, wherein each maintenance dose is equivalent to about 28 mg of the antisense compound. In some cases, the second loading dose is administered about 14 days after administration of the first loading dose, the third loading dose is administered about 14 days after administration of the second loading dose, and the fourth loading dose is administered about 14 days after administration of the third loading dose. In other cases, the second loading dose is administered 14 days after administration of the first loading dose, the third loading dose is administered 14 days after administration of the second loading dose, and the fourth loading dose is administered 14 days after administration of the third loading dose.

In another aspect the disclosure provides a method for treating SMA in a human subject in need thereof. The method involves administering to the human subject an antisense compound having the following structure: 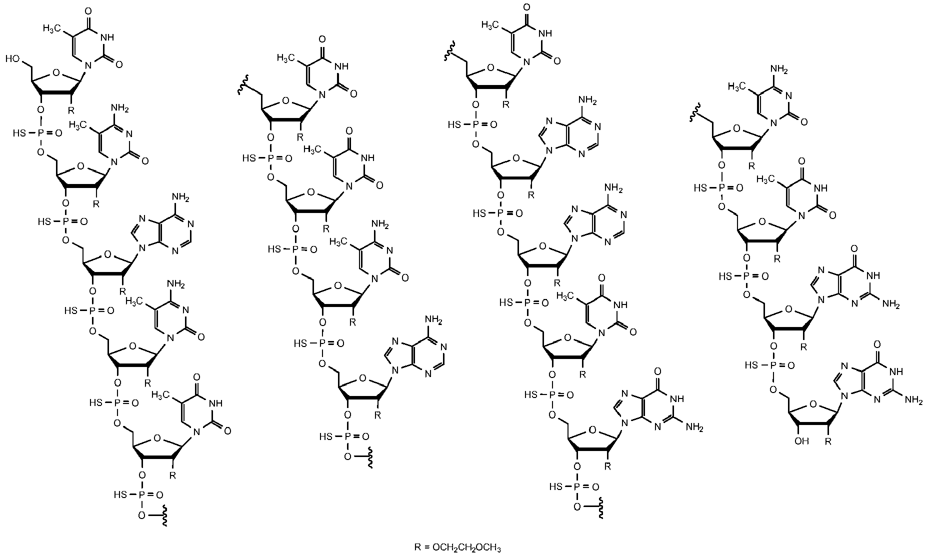 or a salt thereof. The human subject being treated was previously administered one or more doses of the antisense compound or the salt thereof described herein, wherein the one or more previously administered doses were equivalent to about 12 mg of the antisense compound. The method involves administering to the human subject by intrathecal injection (e.g., intrathecal bolus injection) a loading dose of the antisense compound or the salt thereof described herein, that is equivalent to about 50 mg of the antisense compound, administered about 3 to 6 months (e.g., about 4 to 5 months; or about 4 months) after administration of the last previously administered dose equivalent to about 12 mg of the antisense compound; and one or more maintenance doses of the antisense compound or the salt thereof described herein, beginning about 3 to 6 months (e.g., about 4 to 5 months; or about 4 months) after administration of the loading dose and continuing once about every 3 to 6 months (e.g., about 4 to 5 months; or about 4 months) thereafter, wherein each maintenance dose is equivalent to about 28 mg of the antisense compound.

In another aspect, the disclosure relates to a method for increasing levels of SMN2 mRNA containing exon 7 in a human subject having mutations in chromosome 5q that are associated with SMN protein deficiency. The method involves administering to the human subject an antisense compound having the following structure: 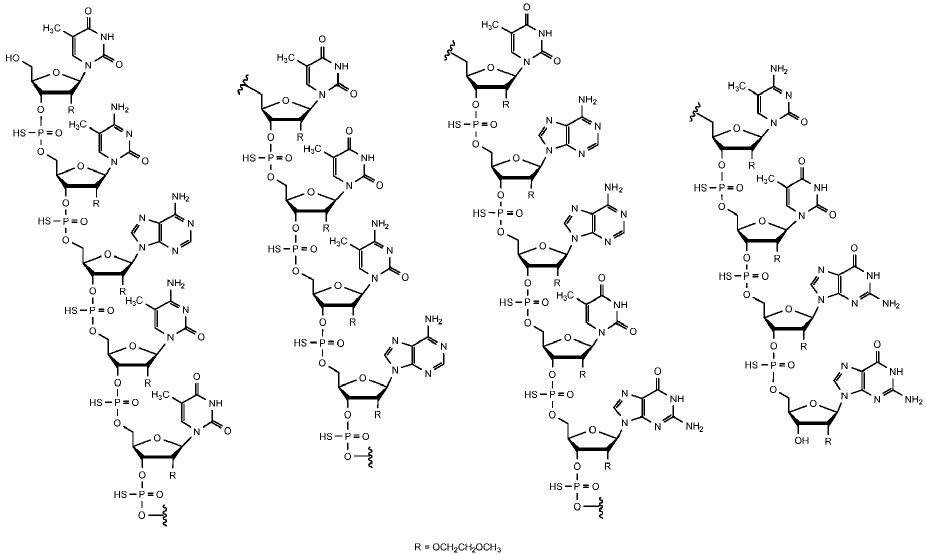 or a salt thereof. The human subject was previously administered one or more doses of the antisense compound or the salt thereof described herein, wherein the one or more previously administered doses were equivalent to about 12 mg of the antisense compound. The method involves administering to the human subject by intrathecal injection (e.g., intrathecal bolus injection) a loading dose of the antisense compound or the salt thereof described that is equivalent to about 50 mg of the antisense compound, administered about 3 to 6 months (e.g., about 4 to 5 months; or about 4 months) after administration of the last previously administered dose equivalent to about 12 mg of the antisense compound; and one or more maintenance doses of the antisense compound or the salt thereof described herein, beginning about 3 to 6 months (e.g., about 4 to 5 months; or about 4 months) after administration of the loading dose and continuing once about every 3 to 6 months (e.g., about 4 to 5 months; or about 4 months) thereafter, wherein each maintenance dose is equivalent to about 28 mg of the antisense compound.

In another aspect, provided is a method for treating spinal muscular atrophy (SMA) in a human subject in need thereof. The method involves administering to the human subject an antisense compound having the following structure: 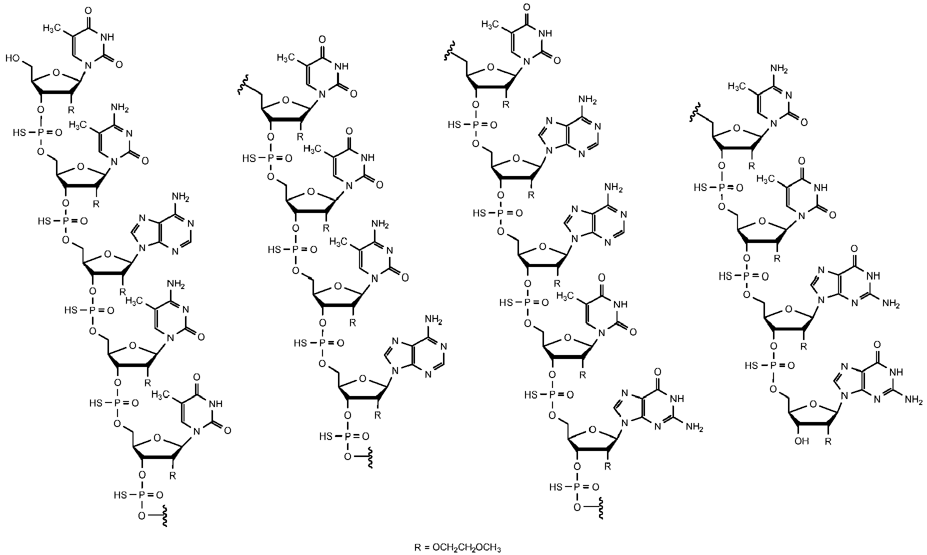 or a salt thereof. The human subject was previously administered one or more doses of the antisense compound or the salt thereof described herein, wherein the one or more previously administered doses were equivalent to about 12 mg of the antisense compound. The method involves administering to the human subject by intrathecal injection (e.g., intrathecal bolus injection): a first loading dose of the antisense compound or the salt thereof described herein that is equivalent to about 28 mg of the antisense compound, administered about 3 to 6 months (e.g., about 4 to 5 months; or about 4 months) after administration of the last previously administered dose equivalent to about 12 mg of the antisense compound; a second loading dose of the antisense compound or the salt thereof described herein that is equivalent to about 50 mg of the antisense compound, administered about 3 to 6 months (e.g., about 4 to 5 months; or about 4 months) after administration of the first loading dose; and one or more maintenance doses of the antisense compound or the salt thereof described herein, beginning about 3 to 6 months (e.g., about 4 to 5 months; or about 4 months) after administration of the second loading dose and continuing once about every 3 to 6 months (e.g., about 4 to 5 months; or about 4 months) thereafter, wherein each maintenance dose is equivalent to about 28 mg of the antisense compound.

In another aspect, the disclosure provides a method for increasing levels of SMN2 mRNA containing exon 7 in a human subject having mutations in chromosome 5q that are associated with SMN protein deficiency. The method comprising administering to the human subject an antisense compound having the following structure: 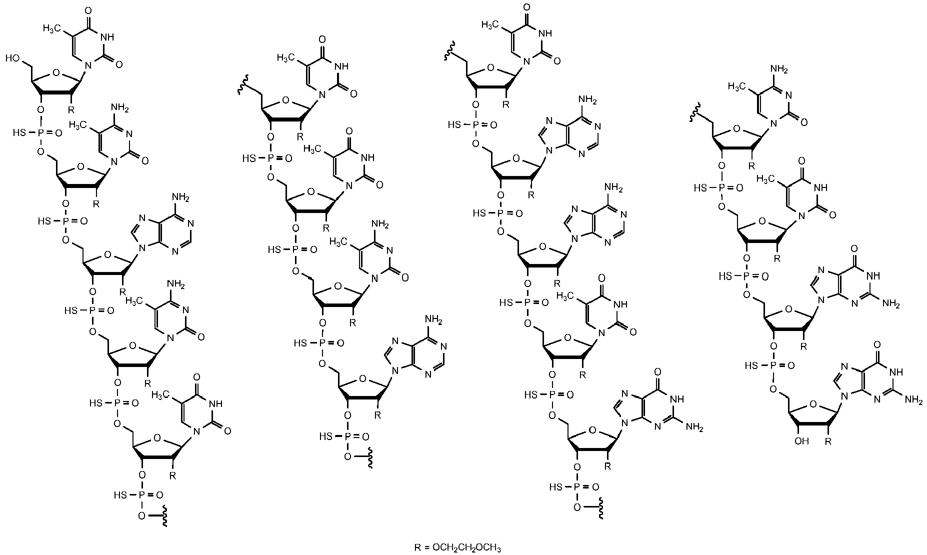 or a salt thereof. The human subject was previously administered one or more doses of the antisense compound or the salt thereof described herein, wherein the one or more previously administered doses were equivalent to about 12 mg of the antisense compound. The method involves administering to the human subject by intrathecal injection (e.g., intrathecal bolus injection): a first loading dose of the antisense compound or the salt thereof described herein that is equivalent to about 28 mg of the antisense compound, administered about 3 to 6 months (e.g., about 4 to 5 months; or about 4 months) after administration of the last previously administered dose equivalent to about 12 mg of the antisense compound; a second loading dose of the antisense compound or the salt thereof described herein that is equivalent to about 50 mg of the antisense compound, administered about 3 to 6 months (e.g., about 4 to 5 months; or about 4 months) after administration of the first loading dose; and one or more maintenance doses of the antisense compound or the salt thereof described herein, beginning about 3 to 6 months (e.g., about 4 to 5 months; or about 4 months) after administration of the second loading dose and continuing once about every 3 to 6 months (e.g., about 4 to 5 months; or about 4 months) thereafter, wherein each maintenance dose is equivalent to about 28 mg of the antisense compound.

In some instances, the human subject was previously administered at least four (e.g., 4, 5, 6, 7, 8, 9, 10), at least five, at least six, at least seven, at least eight, at least nine, or at least ten doses of the antisense compound or the salt thereof described herein, wherein each of the at least four, five, six, seven, eight, nine, or ten previously administered doses was equivalent to about 12 mg of the antisense compound.

In some instances of all the above methods, the human subject has type I SMA; type II SMA; type III SMA; or type IV SMA. In other instances, the human subject is presymptomatic for SMA.

In some instances, the human subject is administered the antisense compound or the salt thereof described herein using a spinal anesthesia needle.

In some instances, the antisense compound or the salt thereof described herein is administered in an injection volume of 5.0 mL.

In some instances, the antisense compound or the salt thereof described herein is dissolved in phosphate buffered saline (PBS). In other instances, the antisense compound or the salt thereof described herein is dissolved in artificial cerebrospinal fluid (aCSF).

In certain instances, the salt of the antisense compound is a sodium salt.

In one instance, the salt of the antisense compound has the following structure:

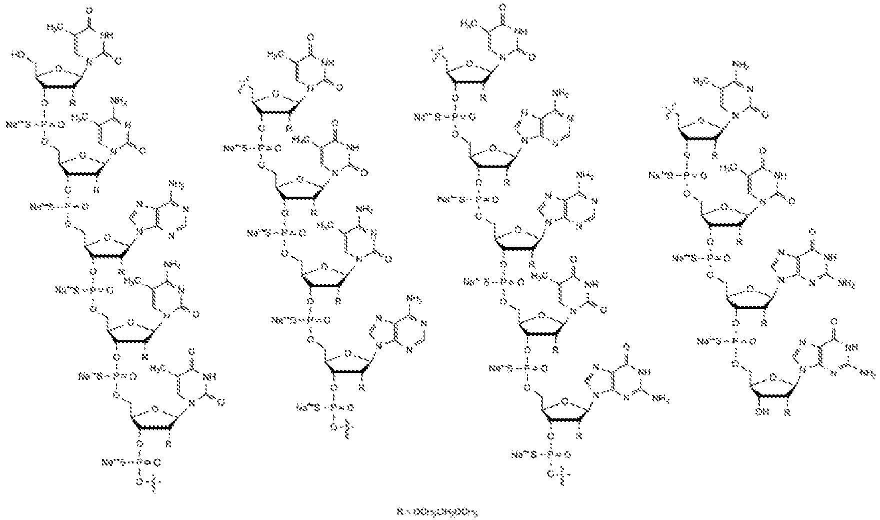

In some embodiments of the methods described herein, the method further comprises a step of detecting a neurofilament level in the human subject before and/or after initiation of treatment. The levels of neurofilament can be used, for example, to determine if a subject administered an antisense compound or salt thereof described herein is responding to the treatment and determine appropriate future treatments for the subject.

In one embodiment, the method entails (i) measuring a first neurofilament level in a first biological sample obtained from the human subject before initiation of treatment; (ii) administering an antisense compound or salt thereof described herein, to the human subject according to any one of the method described herein; and (iii) measuring neurofilament level in a subsequent (e.g., second) biological sample obtained from the human subject after administering at least one loading dose

In one embodiment, the method entails (i) measuring a first neurofilament level in a first biological sample obtained from the human subject before initiation of treatment; (ii) administering an antisense compound or salt thereof described herein, to the human subject according to any one of the method described herein; and (iii) measuring neurofilament level in a subsequent (e.g. second) biological sample obtained from the human subject after administering all loading doses.

In one embodiment, the method entails (i) measuring a first neurofilament level in a first biological sample obtained from the human subject before initiation of treatment; (ii) administering the antisense compound or salt thereof described herein to the human subject according to any one of the method described herein; and (iii) measuring neurofilament level in a subsequent (e.g., second) biological sample obtained from the human subject after administering all loading doses and at least one maintenance dose of the antisense compound or salt thereof described herein.

In one example, the subsequent (e.g., second) neurofilament level is lower than the first neurofilament level (e.g., the second neurofilament level is reduced by at least 50% compared to the first neurofilament level) and the method further comprises administering either another loading dose or another maintenance dose, each as described herein, at the respective dosing intervals, as described herein.

In another example, the subsequent (e.g., second) neurofilament level is equal to or higher than the first neurofilament level and the method further comprises administering further doses of the antisense compound or salt thereof as described herein, to the human subject, wherein each of the further doses of the antisense compound or salt thereof is in an increased amount and/or at a shortened dosing interval as compared to the last dose administered prior to measuring the subsequent (e.g. second) neurofilament level.

In some embodiments, the neurofilament is a neurofilament heavy chain (NF-H, e.g., pNF-H), medium/intermediate chain (NF-M), or light chain (NF-L).

In some embodiments, the first and second biological samples are blood, serum, plasma, or cerebrospinal fluid.

The term "about" in the context of an amount, e.g., about X mg means +/- 10%, so "about 50 mg" encompasses 45 mg to 55 mg. The term "about" in the context of X days means +/-3 days, so "about 10 days" encompasses 7 to 13 days. The term "about" in the context of X months means +/-1 week, so "about 4 months" encompasses a week before and after the 4 month mark.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. All publications, patent applications, patents, and other references mentioned herein are incorporated by reference in their entirety. In case of conflict, the present application, including definitions, will control. The materials, methods, and examples are illustrative only and not intended to be limiting.

Other features and advantages of the invention will be apparent from the following detailed description and from the claims.

### Brief Description of the Drawings

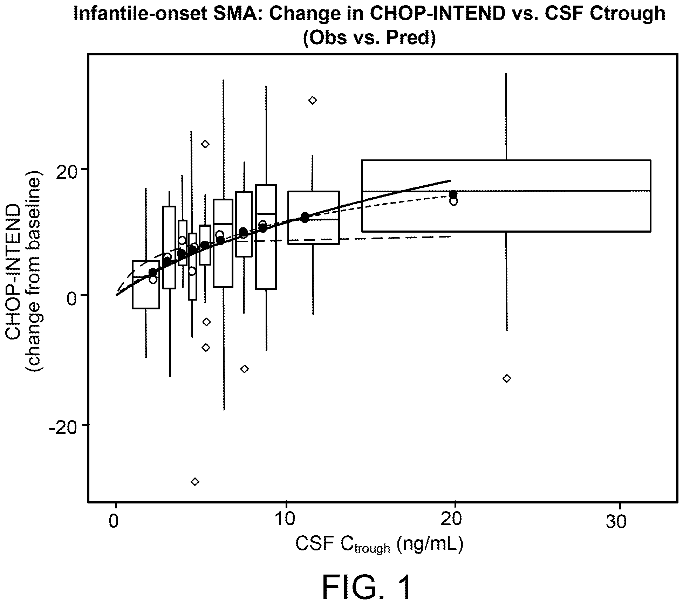

Figure 1 shows the relationship between CSF C_{trough} and CHOP-INTEND (change from baseline) with fixed E0 and Emax (E= efficacy). Dashed (long), solid, and dashed (shorter) line represents the predicted PK/PD relationship when Emax was fixed at 10, 25, and 40, respectively. The observed data are divided based on 10 quantiles of CSF C_{trough} values. The empty and filled circles represent the mean observed and predicted change in CHOP-INTEND from baseline, respectively.

### Detailed Description

This disclosure relates to methods for treating or preventing SMA using effective dosage regimens of an antisense compound or salt thereof, each as described herein. The methods are useful for the treatment and/or prevention of SMA in pediatric and adult patients.

Patients with SMA (Type I, II, III, IV and pre-symptomatic) may achieve greater improvement in motor function and motor milestone development with antisense drug exposure that is higher than the concentrations achieved with the current SPINRAZA^{®} dosing regimen.

### Antisense Compounds

An exemplary antisense compound for use in the invention has the nucleobase sequence: TCACTTTCATAATGCTGG (**SEQ ID NO: 1**), wherein each nucleoside has a 2'-O-(2-methoxyethyl) (MOE) modification; each internucleoside linkage of the oligonucleotide is a phosphorothioate linkage; and each "C" in the sequence is 5-methyl-cytosine.

The free acid form of this antisense compound (also referred to as nusinersen) is depicted below:

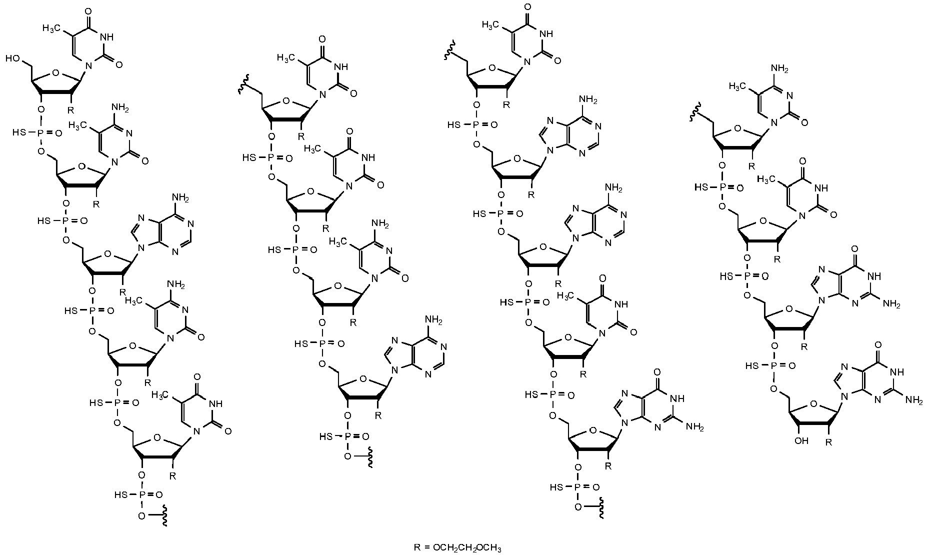

In some instances, a salt of this antisense compound (e.g., a pharmaceutically acceptable salt) is used. In some cases, the salt can be sodium (also referred to as nusinersen sodium or nusinersen sodium salt). In other instances, the salt can be potassium.

An exemplary salt of this antisense compound is depicted below:

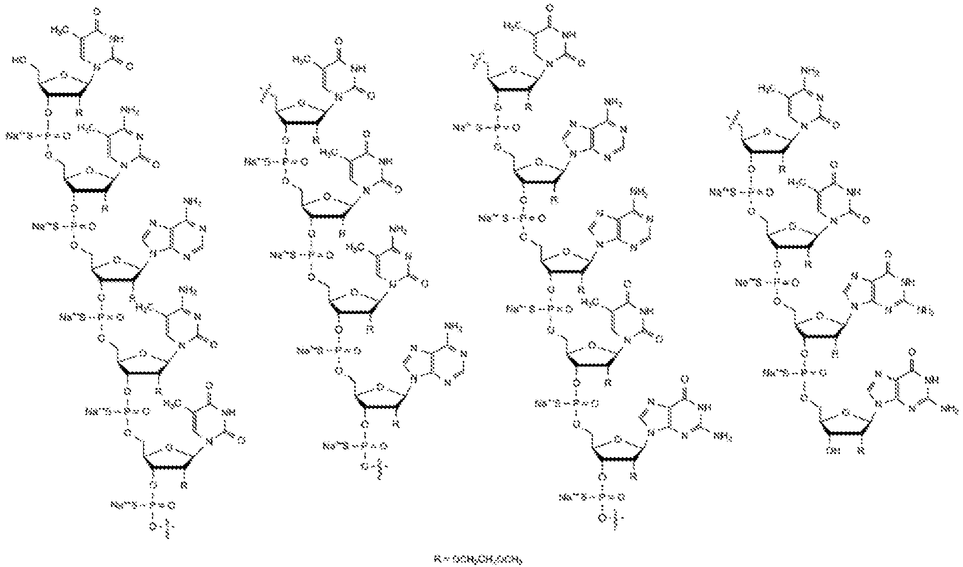

The antisense compound is nusinersen and its sodium salt is nusinersen sodium or nusinersen sodium salt. 2.40 mg of nusinersen is equivalent to 2.53 mg of nusinersen sodium salt. A dose of nusinersen sodium that is equivalent to 50 mg of nusinersen is 52.71 mg of nusinersen sodium salt. A dose of nusinersen sodium that is equivalent to 28 mg of nusinersen is 29.52 mg of nusinersen sodium salt. A dose of nusinersen sodium that is equivalent to 12 mg of nusinersen is 12.65 mg of nusinersen sodium salt.

The antisense compound or salt thereof can be prepared as a solution for injection by intrathecal administration.

The antisense compounds described herein can be used to treat or prevent SMA in a human subject in need thereof. In some instances, the SMA is Type I SMA. In other instances, the SMA is Type II SMA. In certain instances, the SMA is Type III SMA. In some instances, the SMA is Type IV SMA. In other instances, the human subject is presymptomatic for SMA.

### Pharmaceutical Compositions

The present disclosure also provides pharmaceutical compositions comprising an antisense compound or salt thereof described herein. In certain instances, such pharmaceutical compositions comprise or consist of a sterile saline solution and an antisense compound or salt thereof. In some cases, such pharmaceutical compositions are sterile, buffered, isotonic solutions. In some cases, the pharmaceutical compositions are preservative-free.

The antisense compounds or salts thereof described herein may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

Antisense compounds or a salt thereof can be utilized in pharmaceutical compositions by combining such compounds with a suitable pharmaceutically acceptable diluent or carrier. In certain instances, the pharmaceutically acceptable diluent is phosphate-buffered saline (PBS). In certain embodiments, the pharmaceutically acceptable diluent is artificial cerebrospinal fluid (aCSF).

In certain embodiments, the aCSF formulation has a pH of 7.2. The pH of the composition can be adjusted, if necessary, with hydrochloric acid or sodium hydroxide during compounding. In some instances, the pharmaceutical composition comprises an antisense compound or salt thereof (e.g., sodium salt) described herein at a concentration of about 10.0 mg/mL. In certain instances, the pharmaceutical composition comprises an antisense compound or salt thereof (e.g., sodium salt) described herein at a concentration of 10.0 mg/mL. In other instances, the pharmaceutical composition comprises an antisense compound or salt thereof (e.g., sodium salt) described herein at a concentration of about 5.6 mg/mL. In some instances, the pharmaceutical composition comprises an antisense compound or salt thereof (e.g., sodium salt) described herein at a concentration of 5.6 mg/mL. In certain instances, the pharmaceutical composition comprises an antisense compound or salt thereof (e.g., sodium salt) described herein at a concentration of 3.6 mg/mL to 10.0 mg/mL. In other instances, the pharmaceutical composition comprises an antisense compound or salt thereof (e.g., sodium salt) described herein at a concentration of 4.8 mg/mL to 10.0 mg/mL. In yet other instances, the pharmaceutical composition comprises an antisense compound or salt thereof (e.g., sodium salt) described herein at a concentration of 5.6 mg/mL to 10.0 mg/mL.

In some instances, the pharmaceutical composition comprises about 50 mg of an antisense compound or salt thereof (e.g., sodium salt) described herein. In some instances, the pharmaceutical composition comprises 50 mg of an antisense compound or salt thereof (e.g., sodium salt) described herein. In other instances, the pharmaceutical composition comprises about 28 mg of an antisense compound or salt thereof (e.g., sodium salt) described herein. In some instances, the pharmaceutical composition comprises 28 mg of an antisense compound or salt thereof (e.g., sodium salt) described herein. In some instances, the pharmaceutical composition is formulated in PBS or aCSF. In certain instances, the pharmaceutical composition is formulated for intrathecal administration.

In one instance, the disclosure features a pharmaceutical composition comprising 50 mg of an antisense compound or salt thereof disclosed herein in a 5 mL volume of a pharmaceutical excipient (e.g., a CSF, PBS) so that the antisense compound concentration is 10 mg/mL. In certain instances, the pharmaceutical composition is formulated for intrathecal administration (e.g., intrathecal bolus injection).

In another instance, the disclosure features a pharmaceutical composition comprising 28 mg of an antisense compound or salt thereof disclosed herein in a 5 mL volume of a pharmaceutical excipient (e.g., a CSF, PBS) so that the antisense compound concentration is 5.6 mg/mL. In certain instances, the pharmaceutical composition is formulated for intrathecal administration (e.g., intrathecal bolus injection).

Pharmaceutical compositions comprising antisense compounds encompass any pharmaceutically acceptable salts, esters, or salts of such esters. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

### Methods of Treatment

The disclosure features a method of treating or preventing spinal muscular atrophy (SMA) in a human subject. In one instance, the SMA is Type I SMA. In another instance, the SMA is Type II SMA. In yet another instance, the SMA is Type III SMA. In another instance, the SMA is Type IV SMA. In certain instances, the human subject is presymptomatic for SMA.

The disclosure also provides a method of increasing inclusion of exon 7 in SMN2 messenger ribonucleic acid (mRNA) transcripts and production of full-length SMN protein in a human subject having loss of both functional copies of the SMN1 gene.

Also featured is a method for increasing exon 7 inclusion in SMN2 messenger ribonucleic acid (mRNA) transcripts in a human subject having mutations in the SMN1 gene that lead to functional SMN protein deficiency.

In addition, the disclosure relates to a method for increasing levels of SMN2 mRNA containing exon 7 in a human subject having mutations in chromosome 5q that are associated with or lead to SMN protein deficiency. The human subject may have a homozygous gene deletion, a substitution, or be a compound heterozygote.

The methods involve administering to a human subject in need thereof an antisense compound described herein or a salt thereof (e.g., a sodium salt). The human subject may be an adult or a child.

In one instance, the human subject is administered a dose of an antisense compound useful for treating SMA or a pharmaceutically acceptable salt thereof by an injection into the intrathecal space of the human subject. The intrathecal administration can be by lumbar puncture (i.e., spinal tap). The administration can be by bolus injection. In one instance, the administration is by intrathecal bolus injection.

In one instance, the antisense compound that is administered to the human subject comprises or consists of the nucleobase sequence set forth in SEQ ID NO: 1, wherein each intemucleoside linkage of the oligonucleotide is a phosphorothioate linkage, each nucleoside of the oligonucleotide is a 2'-MOE nucleoside, and each C is a 5-methylcytosine.

In certain instances, the first dose of an antisense compound or salt thereof described herein is administered when the subject is less than one week old. In certain instances, the first dose of an antisense compound or salt thereof described herein is administered when the subject is less than one month old. In certain instances, the first dose of an antisense compound or salt thereof described herein is administered when the subject is less than 3 months old. In certain instances, the first dose of an antisense compound or salt thereof described herein is administered when the subject is less than or equal to 6 months old. In certain instances, the first dose of an antisense compound or salt thereof described herein is administered when the subject is greater than 6 months old. In certain instances, the first dose of an antisense compound or salt thereof described herein is administered when the subject is less than one year old. In certain instances, the first dose of an antisense compound or salt thereof described herein is administered when the subject is less than 2 years old. In certain instances, the first dose of an antisense compound or salt thereof described herein is administered when the subject is less than 15 years old. In certain instances, the first dose of an antisense compound or salt thereof described herein is administered when the subject is older than 15 years old. In certain instances, the first dose of an antisense compound or salt thereof described herein is administered when the subject is between 1 to less than 18 years of age. In certain instances, the first dose of an antisense compound or salt thereof described herein is administered when the subject is 18 years or older.

In certain instances, the human subject has a SMN2 copy number of 1. In other instances, the human subject has a SMN2 copy number of 2. In some instances, the human subject has a SMN2 copy number of 3. In other instances, the human subject has a SMN2 copy number of 4. In certain instances, the human subject has a SMN2 copy number of 5.

In some instances, the antisense compounds or salts thereof described herein are administered as an intrathecal bolus injection. In some instances, the antisense compounds or salts thereof described herein are administered as a single intrathecal bolus lumbar puncture injection. In certain embodiments, the volume of the intrathecal bolus lumbar puncture injection is 5 mL.

### Dosing Regimen

This disclosure features enhanced or high dosage regimens of antisense compounds or salts thereof described herein for treatment or prevention of SMA. In some instances, the dose of the antisense compound or salt thereof (e.g., sodium salt) used for treating or preventing SMA is equivalent to greater than 12 mg and less than or equal to 50 mg of the antisense compound described herein. In certain instances, the dose of the antisense compound or salt thereof (e.g., sodium salt) used for treating or preventing SMA is equivalent to is about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, or about 50 mg of the antisense compound described herein. In other instances, the dose of the antisense compound or salt thereof (e.g., sodium salt) used for treating or preventing SMA is equivalent to is 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 mg of the antisense compound described herein. In certain embodiments, the dose is administered (e.g., intrathecally) in a volume of 5 mL (e.g., of aCSF or PBS). In some instances, the dose is administered by a bolus injection. In one instance, the dose is administered by an intrathecal bolus injection.

In some instances, a human subject in need thereof is administered an antisense compound or salt thereof described herein at a dose equivalent to 18 to 50 mg of the antisense compound in a 5mL dose volume. In other instances, a human subject in need thereof is administered an antisense compound or salt thereof described herein at a dose equivalent to 24 to 50 mg of the antisense compound in a 5 mL dose volume. In certain instances, a human subject in need thereof is administered an antisense compound or salt thereof described herein at a dose equivalent to 28 to 50 mg of the antisense compound in a 5mL dose volume. In yet another instance, a human subject in need thereof is administered an antisense compound or salt thereof described herein at a dose equivalent to 50 mg of the antisense compound in a 5mL dose volume. In some instances, the dose of the antisense compound or salt thereof described herein, is administered by a bolus injection. In one instance, the dose of the antisense compound or salt thereof described herein, is administered by an intrathecal bolus injection. In certain cases, the antisense compound or salt thereof described herein, is administered in aCSF or PBS.

The dosing regimen can comprise administering (e.g., intrathecally) to a human subject at least two (e.g., 2, 3, 4, 5), about two, or two loading doses of an antisense compound or salt thereof (e.g., sodium salt) described herein that is used for treating or preventing SMA equivalent to about 50 mg of the antisense compound, followed by maintenance doses of the antisense compound or salt thereof (e.g., sodium salt) described herein used for treating or preventing SMA equivalent to about 28 mg of the antisense compound. In some instances, the loading doses are each equivalent to 50 mg of the antisense compound described herein. In some instances, maintenance doses are each equivalent to 28 mg of the antisense compound described herein. In some instances, the loading doses are administered 10 to 20 days apart (e.g., 10 to 18 days apart, 12 to 16 days apart, biweekly) and the maintenance doses are administered 3 to 6 months (e.g., 4 to 5 months or about 4 months) after the last loading dose and every 3 to 6 months thereafter. In some cases, two loading doses are administered. In some cases, three loading doses are administered. In yet other cases, four loading doses are administered. In certain instances, the loading doses are administered 10 to 18 days apart and the maintenance doses are administered 3 to 6 months after the last loading dose and 3 to 6 months thereafter. In some instances, the loading doses are administered 12 to 16 days apart and the maintenance doses are administered 3 to 6 months after the last loading dose and 3 to 6 months thereafter. In other instances, the loading doses are administered 10 to 20 days apart (e.g., biweekly) and the maintenance doses are administered 3 to 6 months after the last loading dose and 3 to 6 months thereafter. In certain instances, the loading doses are administered 14 days apart and the maintenance doses are administered 3 to 6 months after the last loading dose and 3 to 6 months thereafter. In some instances, the maintenance dose(s) is/are administered 4 to 5 months after the last loading dose and 4 to 5 months thereafter. In some instances, the maintenance dose(s) is/are administered about 4 months after the last loading dose and about 4 months thereafter. In some instances, the maintenance dose(s) is/are administered 4 months after the last loading dose and 4 months thereafter. In some instances, at least two, at least three, at least four, at least five, at least six, at least seven, at least eight, at least nine, at least 10, at least 11, or at least 12 maintenance doses are administered.

In one example, a human subject in need thereof is administered a loading dose of the antisense compound depicted below or a salt thereof (e.g., sodium salt): 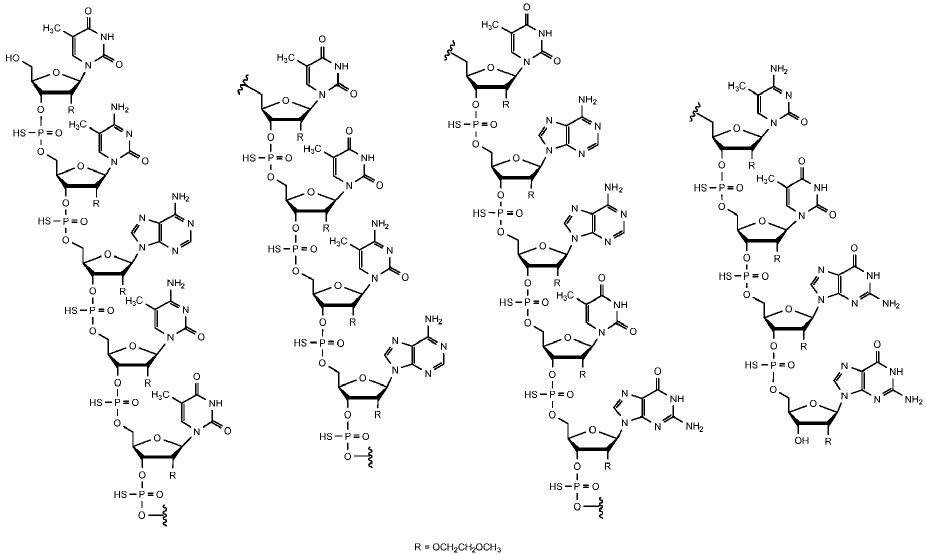 at a dose equivalent to about 50 mg (e.g., 50 mg) of the antisense compound. In some instances, the human subject in need thereof is administered two loading doses of the antisense compound or salt thereof (e.g. sodium salt) described herein, 10-20, 10-18, 12-16, or about 14 days apart, wherein the loading doses are each equivalent to about 50 mg (e.g., 50 mg) of the antisense compound. In some cases, the human subject in need thereof is administered two loading doses of the antisense compound or salt thereof (e.g. sodium salt) described herein, 14 days apart, wherein the two loading doses are each equivalent to about 50 mg (e.g., 50 mg) of the antisense compound. In some cases, the human subject in need thereof is administered two loading doses of the antisense compound or salt thereof (e.g. sodium salt) described herein, biweekly, wherein the two loading doses are each equivalent to about 50 mg (e.g., 50 mg) of the antisense compound. In one instance, the human subject in need thereof is administered, on Day 1, a first loading dose of the antisense compound or salt thereof (e.g. sodium salt) described herein, at a dose equivalent to about 50 mg (e.g., 50 mg) of the antisense compound, and a second loading dose of the antisense compound or salt thereof described herein, on Day 15, equivalent to about 50 mg (e.g., 50 mg) of the antisense compound. In some instances, the human subject is further administered at least one maintenance dose of the antisense compound or salt thereof described herein, wherein the maintenance dose is administered 3 to 6 months, 4 to 5 months, or about 4 months after the administration of the second loading dose. In certain instances, the human subject is further administered one or more additional maintenance doses of the antisense compound or salt described herein, thereof 3 to 6 months, 4 to 5 months, or about 4 months after the administration of the first maintenance dose. In certain instances, the human subject is further administered one or more additional maintenance doses of the antisense compound or salt thereof described herein, 4 months after the administration of the first maintenance dose.

In another example, a human subject in need thereof is administered a loading dose of the salt of an antisense compound that is depicted below: 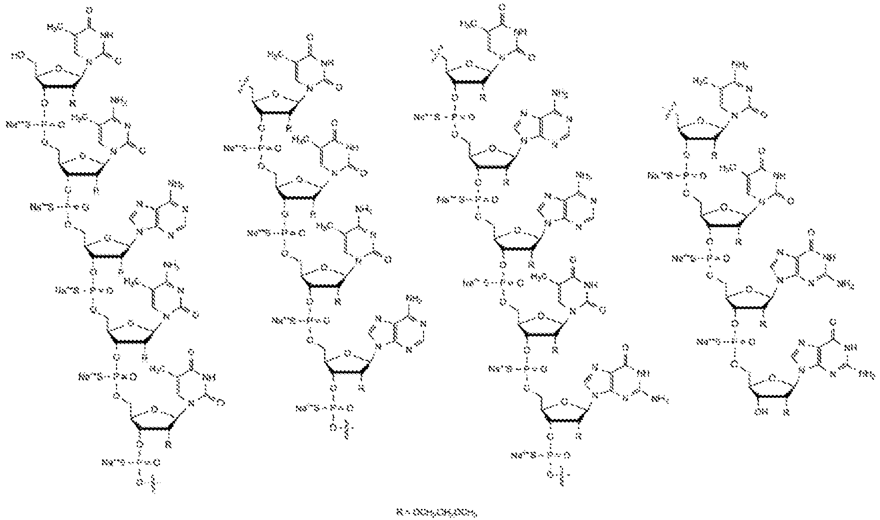 at a dose equivalent to about 50 mg (e.g., 50 mg) of the antisense compound. In some instances, the human subject in need thereof is administered two loading doses of the salt, 10-20, 10-18, 12-16, or about 14 days apart, wherein the loading doses are each equivalent to about 50 mg (e.g., 50 mg) of the antisense compound. In some cases, the human subject in need thereof is administered two loading doses of the salt of the antisense compound, 14 days apart, wherein the two loading doses are each equivalent to about 50 mg (e.g., 50 mg) of the antisense compound. In some cases, the human subject in need thereof is administered two loading doses of the salt of the antisense compound, biweekly, wherein the two loading doses are each equivalent to about 50 mg (e.g., 50 mg) of the antisense compound. In one instance, the human subject in need thereof is administered, on Day 1, a first loading dose of the salt of the antisense compound at a dose equivalent to about 50 mg (e.g., 50 mg) of the antisense compound, and a second loading dose of the salt of the antisense compound, on Day 15, equivalent to about 50 mg (e.g., 50 mg) of the antisense compound. In some instances, the human subject is further administered at least one maintenance dose of the salt of the antisense compound, wherein the maintenance dose is administered 3 to 6 months, 4 to 5 months, or about 4 months after the administration of the second loading dose. In certain instances, the human subject is further administered one or more additional maintenance doses of the salt of the antisense compound 3 to 6 months, 4 to 5 months, or about 4 months after the administration of the first maintenance dose.

In one aspect, a human subject in need thereof (e.g., having SMA (any of types I to IV) or presymptomatic) is administered a first loading dose of an antisense compound or salt thereof described herein on Day 1 at a dose equivalent to about 50 mg (e.g., 50 mg) of the antisense compound, a second loading dose of an antisense compound or salt thereof described herein on about Day 15 at a dose equivalent to about 50 mg (e.g., 50 mg) of the antisense compound, a first maintenance dose of an antisense compound or salt thereof described herein on about Day 135 at a dose equivalent to about 28 mg (e.g., 28 mg) of the antisense compound, and one or more further maintenance doses of an antisense compound or salt thereof described herein about 120 days after the first maintenance dose at a dose equivalent to about 28 mg (e.g., 28 mg) of the antisense compound. In one instance, a human subject in need thereof (e.g., having SMA (any of types I to IV) or presymptomatic) is administered a first loading dose of an antisense compound or salt thereof described herein on Day 1 at a dose equivalent to 50 mg of the antisense compound, a second loading dose of an antisense compound or salt thereof described herein on Day 15 at a dose equivalent to 50 mg of the antisense compound, a first maintenance dose of an antisense compound or salt thereof described herein on any one of Days 134-140 at a dose equivalent to 28 mg of the antisense compound, and one or more further maintenance doses of an antisense compound or salt thereof described herein 253 to 259 days after the first maintenance dose at a dose equivalent to 28 mg of the antisense compound. In some embodiments, the antisense compound has the following structure: 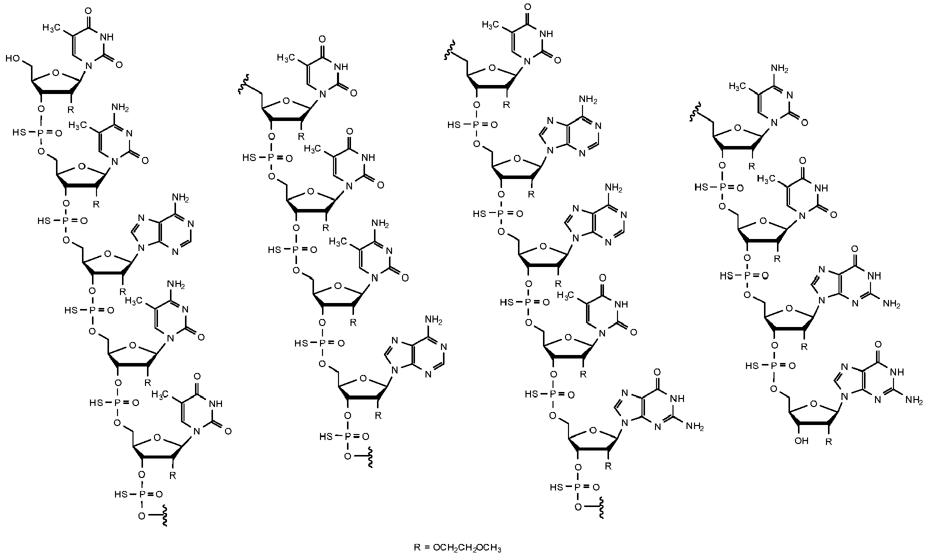 In some embodiments, the salt of the antisense compound has the following structure:

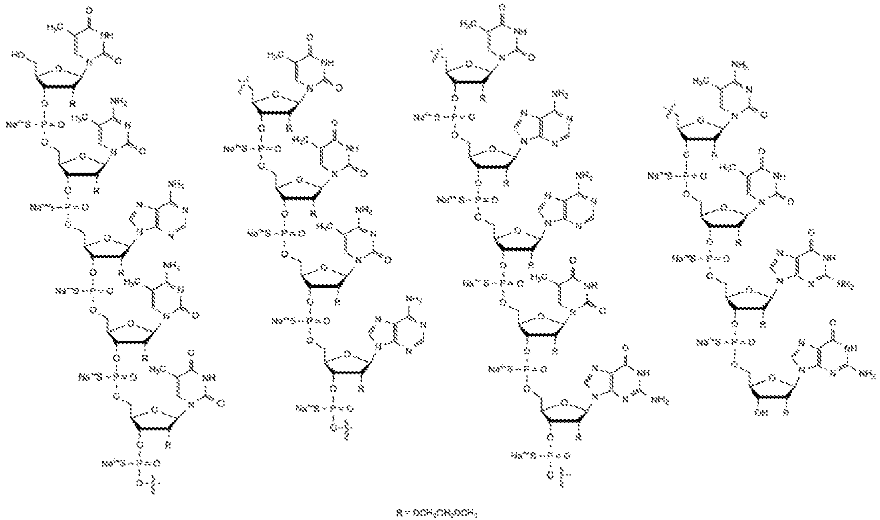

In another aspect, the disclosure features a titration dosing regimen for a human subject in need thereof. This titration (bridging) regimen allows for effectively transitioning subjects on the approved 12 mg regimen of the antisense compound or salt thereof described herein to a regimen that allows increased CSF exposure. In one instance, the titration dosing regimen involves administering to a subject who has previously been administered the antisense compound or salt thereof described herein at a dose equivalent to 12 mg of the antisense compound, a loading dose of the antisense compound or salt thereof described herein equivalent to about 50 mg of the antisense compound. In some cases, the loading dose is a single bolus intrathecal injection. In some instances, the 50 mg loading dose is administered four months after the last 12 mg administration of the antisense compound or salt thereof described herein. In some cases, the 50 mg dose is administered after a 12 mg dose followed by a 28 mg dose. In certain instances, one or more maintenance doses of the antisense compound or salt thereof described herein are administered to the subject at a dose equivalent to 28 mg of the antisense compound beginning about 4 months after the loading dose. In some instances, the subject has had at least one, at least two, at least three, at least four, at least five, or one, two, three, four, or five doses of the antisense compound or salt thereof described herein equivalent to about 12 mg of the antisense compound prior to receiving the loading dose. In some embodiments, the antisense compound has the following structure: 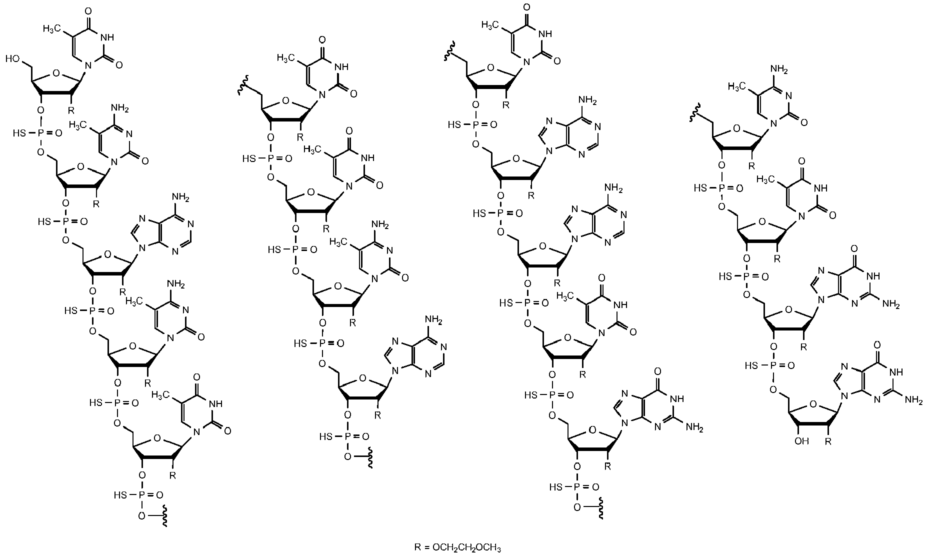 In some instances, the salt of the antisense compound has the following structure:

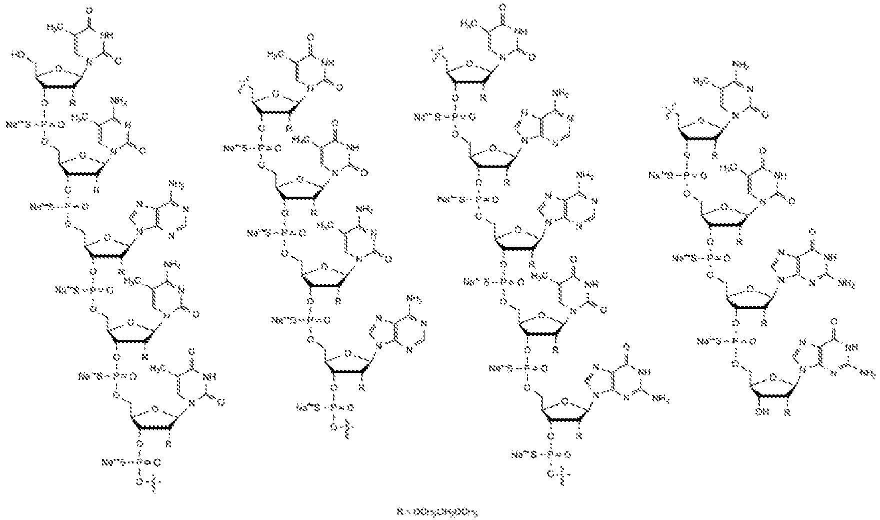

The doses described herein are generally provided in a volume of about 5.0 mL. In one instance, the dose volume is 5.0 mL. In some cases, the diluent is PBS. In other instances, the diluent is aCSF. In certain cases, the loading dose is provided to the human subject at a concentration of 10.0 mg/mL. In some cases, the maintenance dose is provided to the human subject at a concentration of 5.6 mg/mL.

The doses are generally administered intrathecally, e.g., by lumbar puncture. The doses are generally administered as an intrathecal injection (e.g., intrathecal bolus injection).

### Biomarkers

Biomarkers of SMA with diagnostic, prognostic, predictive, and pharmacodynamic value are helpful to facilitate the timing of treatment initiation, determining response to intervention, determining treatment effects, and assessing long-term outcomes. Such biomarkers include genetic, epigenetic, proteomic, electrophysiological, and imaging measures. This disclosure features the use of such biomarkers to facilitate detection of disease states when compared to healthy populations, provide information on likely health outcomes, disease evolution or reoccurrence risk, facilitate stratification of phenotypic severity, identify likely responders to treatment and patient populations, confirm response to therapy, and/or monitor therapeutic efficacy.

In one embodiment, the biomarker is a neurofilament protein. Neurofilaments are the predominant cystokeletal element in nerve cells and play a role not only in conferring mechanical stability but also in determining axonal caliber. Human neurofilaments are composed of three protein subunits, neurofilament light chain (NF-L), neurofilament medium/intermediate chain (NF-M), and neurofilament heavy chain (NF-H). These proteins share the same basic architecture as other intermediate filament subunit proteins. Neurofilaments in the mammalian nervous system also contain the protein intemexin and neurofilaments in the peripheral nervous system can also contain the protein peripherin. Thus, as used herein, by "a neurofilament protein" is meant NF-H, NF-M, NF-L, internexin, or peripherin. The SMA biomarker can be one or more of NF-H, NF-M, NF-L, internexin, and peripherin. In certain instances, the SMA biomarker is a phosphorylated NF-H (pNF-H). In certain instances, the SMA biomarker is a phosphorylated NF-L.

In nusinersen clinical trials with SMA types 1, 2 and presymptomatic infants, plasma pNf-H differentiated SMA individuals from healthy controls. Treatment initiation with nusinersen was associated with rapid decline followed by stabilization of pNf-H at levels close to those of healthy controls. Thus assessing the levels of neurofilament (e.g., pNf-H and/or NfL) can be a helpful biomarker prior or during treatment.

The levels of neurofilament can be used to determine if a subject (e.g., human) administered an antisense compound or salt thereof described herein is responding to the treatment. This can be assessed by obtaining a first biological sample from the subject before and a second biological sample after administering the treatment to the subject and measuring the level of neurofilament (e.g., NF-H, NF-M, or NF-L) in such samples. In one instance, the level of neurofilament is a level of pNF-H.

In certain instances, the first biological sample or samples can be collected from the subject (e.g., human) any time before treatment, e.g., a week before, several days before, a day before, several hours before, an hour before, or less than an hour before, administering the antisense compound or salt thereof described herein. Similarly, the second biological sample or samples can be collected from the subject any time after administration of the treatment, e.g., less than an hour after, an hour after, several hours after, a day after, several days after, a week after, several weeks after, a month after, two months after, three months after, four months after, five months after, 6 months after, 7 months after, or 8 months after, administering the antisense compound or salt thereof described herein. A reduction in neurofilament level after commencing treatment is indicative of the effectiveness of the treatment. In such instances, continuation of the treatment therapy is indicated (e.g., administering further doses of the antisense compound in the same or lesser amount as the last dose administered prior to measuring the second neurofilament level and at the same or lengthened dosing interval as compared to the last dose administered prior to measuring the second neurofilament level). Failure to reduce neurofilament level after commencing treatment is indicative of the need for altering the dose and/or dosing interval of the treatment (e.g., administering further doses of the antisense compound in an increased amount and/or at a shortened dosing interval as compared to the last dose administered prior to measuring the second neurofilament level) or the lack of effectiveness of the treatment. In the latter instance, discontinuation of the treatment may be suggested and the use of a different SMA therapy or therapies is to be considered.

The level of neurofilament can be assessed by measuring RNA or protein levels. In some instances, the level of pNF-H is determined. The concentration of the neurofilament protein or proteins of interest can be measured using any method known in the art such as an immunological assay. Non-limiting examples of such methods include enzyme immunoassay, radioimmunoassay, chemiluminescent immunoassay, electrochemiluminescence immunoassay, latex turbidimetric immunoassay, latex photometric immunoassay, immunochromatographic assay, and western blotting. In certain embodiments, the concentration of the protein or proteins of interest is measured by mass spectrometry.

In certain instances, a neurofilament level (e.g., pNF-H) in the first biological sample is above 300 pg/mL, above 400 pg/mL, above 500 pg/mL, above 600 pg/mL, above 700 pg/mL, above 800 pg/mL, above 900 pg/mL, above 1,000 pg/mL, above 1,500 pg/mL, above 2,000 pg/mL, above 3,000 pg/mL, above 4,000 pg/mL, or above 5,000 pg/mL. In certain embodiments, the neurofilament level measured in a subsequent (e.g., second) biological sample is lower than the neurofilament level measured in the first biological sample. In some embodiments, the neurofilament level measured in a subsequent (e.g., second) biological sample shows a greater than 30% (e.g., greater than 31%, 35%, 40%, 45%, 50%, 55%, 60%, 65%, 70%, 80%, 85%, 90%, or 95%) decline relative to the neurofilament level measured in the first biological sample.

In another embodiment, the SMA biomarker is any one of those described in WO2011/032109.

In another embodiment, the SMA biomarker is any one of those described in WO2019197954.

In another embodiment, the SMA biomarker is any one of those described in WO2019/122125.

In another embodiment, the SMA biomarker is any one of those described in WO2019/147960.

In yet another embodiment the SMA biomarker is any one or more of COMP, DPP4, SPP1, CLEC3B, VTN, and AHSG.

In some embodiments, a combination(s) of any of the biomarkers discussed above are assayed from a sample of the subject (e.g., human).

### Kits

This disclosure also features kits for treating spinal muscular atrophy comprising the antisense compound or salt thereof (e.g., sodium salt) described herein. In some instances, the kit comprises at least one pharmaceutical composition that comprises about 50 mg of an antisense compound or salt thereof (e.g., sodium salt) described herein and at least one pharmaceutical composition that comprises about 28 mg of an antisense compound or salt thereof (e.g., sodium salt) described herein. In some instances, the kit comprises at least one pharmaceutical composition that comprises 50 mg of an antisense compound or salt thereof (e.g., sodium salt) described herein and at least one pharmaceutical composition that comprises 28 mg of an antisense compound or salt thereof (e.g., sodium salt) described herein. In some instances, the kit comprises a pharmaceutical composition that comprises about 50 mg of an antisense compound or salt thereof (e.g., sodium salt) described herein. In some instances, the kit comprises a pharmaceutical composition that comprises 50 mg of an antisense compound or salt thereof (e.g., sodium salt) described herein. In other instances, the kit comprises a pharmaceutical composition that comprises about 28 mg of an antisense compound or salt thereof (e.g., sodium salt) described herein. In some instances, the kit comprises a pharmaceutical composition that comprises 28 mg of an antisense compound or salt thereof (e.g., sodium salt) described herein. In some instances, the pharmaceutical composition is formulated in PBS or aCSF. In some instances, the kit comprises an antisense compound or salt thereof (e.g., sodium salt) described herein is diluted in 5 mL of PBS or aCSF. In some instances, the kit comprises an antisense compound or salt thereof (e.g., sodium salt) described herein at a concentration of 10 mg/mL. In some instances, the kit comprises an antisense compound or salt thereof (e.g., sodium salt) described herein at a concentration of 5.6 mg/mL In certain instances, the kit comprises a pharmaceutical composition formulated for intrathecal administration. In some instances, the kit comprises a pharmaceutical composition formulated for intrathecal bolus injection.

In one instance, the kit can comprise a lyophilized form of an antisense compound or salt thereof (e.g., sodium salt) described herein, a volume (e.g., 5 to 10 mL) of diluent (e.g., PBS, aCSF), a syringe for injection, and/or instructions for use.

In one instance, the kit can comprise at least one single-dose vial containing 5 mL of a pharmaceutical composition, wherein the pharmaceutical composition comprises 50 mg of an antisense compound or salt thereof described herein, so that the antisense compound concentration in the single-dose vial is 10 mg/mL.

In one instance, the kit can comprise at least one single-dose vial containing 5 mL of a pharmaceutical composition, wherein the pharmaceutical composition comprises 28 mg of an antisense compound or salt thereof described herein, so that the antisense compound concentration in the single-dose vial is 5.6 mg/mL. In some instances, the kit as described herein further comprises a syringe for injection and/or instructions for use.

In one instance, the kit can comprise (i) at least one single-dose vial containing 5 mL of a pharmaceutical composition, wherein the pharmaceutical composition comprises 50 mg of an antisense compound or salt thereof described herein, so that the antisense compound concentration in the single-dose vial is 10 mg/mL; and (ii) at least one single-dose vial containing 5 mL of a pharmaceutical composition, wherein the pharmaceutical composition comprises 28 mg of an antisense compound or salt thereof described herein, so that the antisense compound concentration in the single-dose vial is 5.6 mg/mL. In some instances, the kit as described herein further comprises a syringe for injection and/or instructions for use.

The following examples are provided to better illustrate the claimed invention and are not to be interpreted as limiting the scope of the invention. To the extent that specific materials are mentioned, it is merely for purposes of illustration and is not intended to limit the invention. One skilled in the art can develop equivalent means or reactants without the exercise of inventive capacity and without departing from the scope of the invention.

### Examples

### Example 1: Nusinersen Population PK/PD Analyses

In the nusinersen clinical studies in subjects with SMA, subjects who received treatment before or shortly after SMA symptom onset and diagnosis generally had better outcomes than subjects with longer disease duration at the time of treatment initiation. In the infantile-onset SMA study, the majority of deaths among nusinersen-treated infants occurred within the first 2 months of the study, before the completion of the loading dose regimen and the achievement of steady-state nusinersen concentrations. Given the rapid decline associated with Type I SMA and the importance of early treatment, the more rapid achievement of higher nusinersen concentrations may further enhance the efficacy of nusinersen and prevent or further mitigate disease progression in patients with SMA. Results from the infantile-onset SMA study along with results from the sham controlled study in subjects with later onset SMA support the greater benefit conferred by achieving the therapeutic concentration earlier in the disease course, as subjects in both studies who had shorter disease duration at the time of study enrollment had better outcomes than subjects with longer disease duration.

### Example 2: Exposure-Response Relationship

During the nusinersen clinical development program, an exploratory exposure-response analysis was conducted using data for 14 subjects with infantile-onset SMA who received nusinersen. Results of this analysis demonstrated a statistically significant positive correlation between nusinersen CSF exposure and motor function. A dose-related trend was observed in the attainment of motor milestones in subjects, with earlier and greater improvement in subjects who received 12 mg loading doses as compared to subjects who received 6 mg loading doses on Days 1, 15, and 85. All subjects received maintenance doses of 12 mg every 4 months after Day 85. Separation of the response curves for the 2 cohorts began around Day 29 and became wider over the course of the study.

Greater improvement in motor function was also observed in subjects with infantile-onset SMA who received more frequent dosing (12 mg loading doses on Days 1, 15, 29, and 64, and 12 mg maintenance doses every 4 months).

In studies to explore the relationship between nusinersen exposure and clinical response, data from subjects with infantile-onset SMA (subjects <6 months of age at diagnosis or symptom onset) were used. Analyses were conducted to characterize a quantitative PK/PD relationship for nusinersen and to determine if higher nusinersen exposure is associated with additional efficacy in the infantile-onset SMA population. Overall, the PK/PD relationship between the observed CSF C_{trough} and the motor function efficacy endpoint (change from baseline in CHOP-INTEND) appeared to follow an Emax relationship (**Figure 1**).

Projections of the model suggest that a CSF C_{trough} of 23 ng/mL will achieve 90% of the maximum observed response. This estimated concentration is approximately 2-fold higher than the observed CSF C_{trough} (~10 ng/mL) achieved at steady state with the approved dosing regimen of 12 mg. These results suggest that increased CNS exposure (up to 20 ng/mL) can produce clinical efficacy above the levels observed in the clinical studies conducted to date. Additionally, this higher clinical exposure is supported by existing clinical safety and tolerability observed in other studies (maximum mean [SD] CSF C_{trough} of 31.3 [24.8] ng/mL) as well as preclinical safety results in non-human primates (data not shown).

The PK/PD relationship described above has been demonstrated primarily in the infantile-onset SMA population, due to limited data and lower drug exposure from the less frequent dosing regimen used in the later-onset population (~4.77 ng/mL on Study Day 273). However, subjects with later-onset SMA who received the approved (more frequent) dosing regimen had similar CSF C_{trough} concentrations (~10 ng/mL on Day 302) as subjects with infantile-onset SMA within the same study (10.7 ng/mL on Day 302). It is expected that the positive PK/PD relationship observed in subjects with infantile-onset SMA would be the same across SMA types and patient age groups who share a similar mechanism of disease and drug action. This is supported by the preliminary analysis from another study, which showed a positive correlation between CSF C_{trough} and total motor milestones scores in subjects with infantile- or later-onset SMA who received 12 mg of nusinersen as 4 loading doses, followed by maintenance doses of 12 mg every 4 months (data not shown).

### Example 3: Loading Dose Optimization

PK simulations were conducted with the goal of identifying loading dosing regimens that will produce the targeted higher drug exposure (approximate 2-fold increase) more rapidly with fewer intrathecal (IT) doses. These simulations were performed for both infantile- and later-onset SMA populations following 2 years of treatment and were based on a population PK model developed with data from patients between the ages of ≤6 months to 18 years with infantile onset, later-onset, or presymptomatic SMA. Because previous modeling indicated that a 4-month dosing frequency best maintained the CSF concentration achieved at steady state, evaluation of the maintenance dosing frequency was not performed.

Using 20 ng/mL as the clinical CSF C_{trough} target concentration and the predicted CSF PK profiles from 24 mg nusinersen (4 loading doses followed by maintenance doses every 4 months) as the reference dosing regimen, simulations were performed to evaluate additional dosing scenarios with higher doses and reduced loading-dose frequency. As noted above, additional evaluation of the maintenance dosing frequency was not performed, as previous modeling showed that a dosing frequency of every 4 months best maintained the CSF concentration achieved at steady state. Assuming PK linearity, PK simulations were performed in both infantile- and later-onset SMA populations following 2 years of treatment using a population PK model developed from patients across the age range of ≤6 months to 18 years old. Relative to the reference dosing regimen, both 28 mg administered as 3 loading doses (biweekly) and 50 mg administered as 2 loading doses (biweekly), each followed by maintenance doses of 28 mg every 4 months, were identified to achieve the desired CSF C_{trough} (approximately 20 ng/mL) more rapidly at the end of the loading dose period. Nusinersen 28 mg administered as 3 loading doses (biweekly) had a comparable predicted CSF maximum concentration (Cmax) to the reference dosing regimen, whereas the 50 mg dosing regimen surprisingly surpassed the predicted Cmax from the reference dosing regimen. Nonhuman primate safety studies evaluating the safety of both 28 and 50 mg have been conducted and support the use of these doses.

### Example 4: Exemplary Dosing Regimens

- A first loading dose of nusinersen sodium to be administered at a dose that is equivalent to 50 mg of nusinersen;
- A second loading dose of nusinersen sodium to be administered at a dose that is equivalent to 50 mg of nusinersen, wherein the second loading dose is to be administered 10 to 20 days; 10 to 18 days; 12 to 16 days; or 14 days after administration of the first loading dose;
- Optionally, a third and/or fourth loading dose of nusinersen sodium at a dose that is equivalent to 50 mg of nusinersen, wherein the third /fourth loading dose is to be administered 10 to 20 days; 10 to 18 days; 12 to 16 days; or 14 days after administration of the second or third loading dose;
- One or more maintenance doses of nusinersen sodium to be administered at a dose that is equivalent to 28 mg of nusinersen, wherein the one or more maintenance doses is to be administered beginning about 3 to 6 months; 4 to 5 months; or 4 months after administration of the final loading dose and continuing once about every 3 to 6 months, 4 to 5 months, or 4 months thereafter.

Dose Volume: 5 mL
Diluent: aCSF or PBS
Administration: Intrathecal Bolus Injection

### Example 5: Exemplary Dosing Regimen

An exemplary enhanced or high dosage regimen for the antisense compounds or salt thereof described herein for use in human subjects in need thereof (e.g., subjects with infantile-onset SMA, later-onset SMA, adult-onset SMA, presymptomatic for SMA) is provided below:
Loading Dose: a dose of nusinersen sodium that is equivalent to 50 mg of nusinersen to be administered on Days 1 and 15;
Maintenance Dose: a dose of nusinersen sodium that is equivalent to 28 mg of nusinersen to be administered starting 4 months after the second loading dose and every 4 months thereafter.
Dose Volume: 5 mL
Vehicle: aCSF or PBS
Administration: Intrathecal Bolus Injection

### Example 6: Titration (Bridging) Dosing Regimen

Potential titration dosing regimens were also evaluated using PK modeling and simulations to identify a process for effectively transitioning subjects already on the approved 12 mg dosing regimen for nusinersen. The goal of these evaluations is to increase CSF exposure in these subjects to the target concentrations (approximately 20 ng/mL) produced by the enhanced or high dosing regimen.

PK simulations evaluated various dosing regimens with the goal of identifying a titration scheme that does not require an additional loading dose period (maintains the 4-monthly maintenance dosing interval) and achieves the higher target C_{trough} more rapidly without increasing risks to patients. All simulations were performed using the updated population PK model. The titration dosing regimens were simulated following 1-year of treatment with the approved dosing regimen of nusinersen at 12 mg, which included 4 loading doses of 12 mg (Days 1, 15, 29 and 64) and 2 maintenance doses to be administered every 4 months after. All titration dosing regimens are to follow a 4-month dosing frequency to be consistent with the 4-month maintenance dosing frequency of the approved 12 mg dosing regimen.

An exemplary titration dosage regimen (that can increase CSF exposure in a subject to the target concentrations of approximately 20 ng/mL) for the antisense compounds described herein (e.g., nusinersen or sodium salt thereof) for use in human subjects in need thereof (e.g., subjects with infantile-onset SMA, later-onset SMA, adult-onset SMA, presymptomatic for SMA) who have been previously administered 12 mg of nusinersen is provided below:
Loading Dose: a dose of nusinersen sodium that is equivalent to 50 mg of nusinersen to be administered on Day 1;
Maintenance Dose: a dose nusinersen sodium that is equivalent to 28 mg of nusinersen to be administered starting four months after the loading dose and to be administered every four months thereafter.
Dose Volume: 5 mL
Vehicle: aCSF or PBS
Administration: Intrathecal Bolus Injection

### Example 7: Other Titration Regimens

### Regimen 1

- Four months after the last dose of nusinersen sodium equivalent to 12 mg of nusinersen, the patient is to be administered a loading dose of nusinersen sodium equivalent to 50 mg of nusinersen; and
- One or more maintenance doses of nusinersen sodium equivalent to 28 mg of nusinersen is to be administered about once every four months afterwards.

Dose Volume: 5 mL
Vehicle: aCSF or PBS
Administration: Intrathecal Bolus Injection

### Regimen 2

- Four months after the last dose of nusinersen sodium equivalent to 12 mg of nusinersen, the patient is to be administered a first loading dose equivalent to of 28 mg of nusinersen; and
- A second loading dose of nusinersen sodium equivalent to 50 mg of nusinersen is to be administered about four months after the first loading dose; and
- One or more maintenance doses of nusinersen sodium equivalent to 28 mg of nusinersen is to be administered about every four months afterwards.

Dose Volume: 5 mL
Vehicle: aCSF or PBS
Administration: Intrathecal Bolus Injection

### Other Embodiments

While aspects of the invention has been described in conjunction with the detailed description thereof, the foregoing description is intended to illustrate and not limit the scope of the invention, which is defined by the scope of the appended claims. Other aspects, advantages, and modifications are within the scope of the following claims.

In view of the foregoing, it will be appreciated that the invention described herein inter alia relates to the following items:
1. A method for treating spinal muscular atrophy (SMA) in a human subject in need thereof, the method comprising administering to the human subject at least two loading doses of an antisense compound having the following structure: 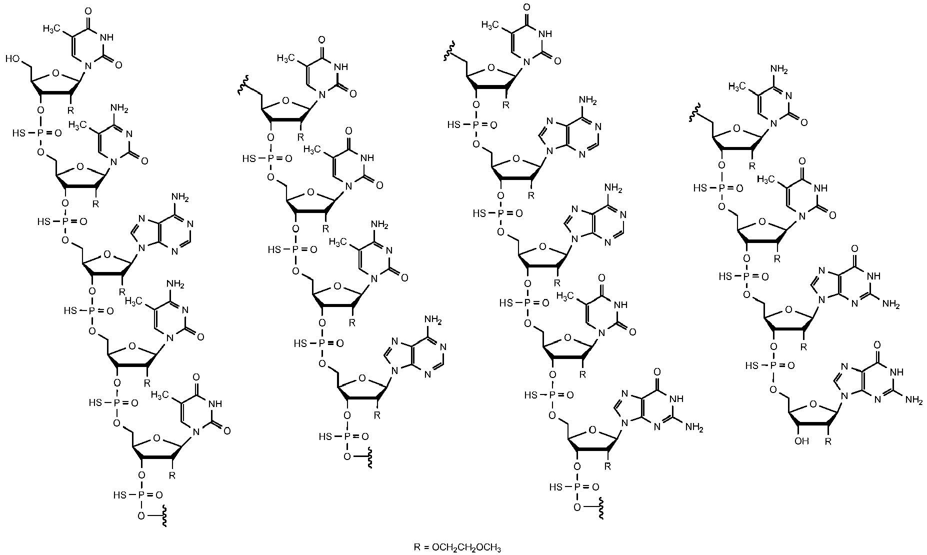 or a salt thereof,
   wherein the method comprises administering to the human subject by intrathecal injection:
   (i) a first loading dose of the antisense compound or the salt thereof that is equivalent to about 50 mg of the antisense compound; and
   (ii) a second loading dose of the antisense compound or the salt thereof that is equivalent to about 50 mg of the antisense compound, administered about 10 to 20 days after administration of the first loading dose.
2. A method for increasing levels of survival motor neuron-2 (SMN2) messenger ribonucleic acid (mRNA) containing exon 7 in a human subject having mutations in chromosome 5q that are associated with SMN protein deficiency, the method comprising administering to the human subject at least two loading doses of an antisense compound having the following structure: 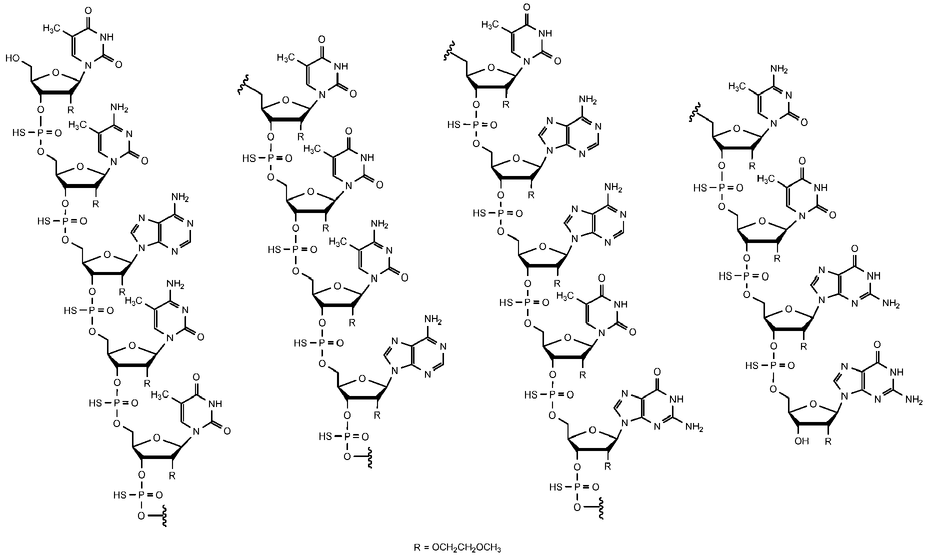 or a salt thereof,
   wherein the method comprises administering to the human subject by intrathecal injection:
   (i) a first loading dose of the antisense compound or the salt thereof that is equivalent to about 50 mg of the antisense compound; and
   (ii) a second loading dose of the antisense compound or the salt thereof that is equivalent to about 50 mg of the antisense compound, administered about 10 to 20 days after administration of the first loading dose.
3. The method of item 1 or 2, further comprising administering to the human subject by intrathecal injection at least one maintenance dose, wherein the at least one maintenance dose is equivalent to about 28 mg of the antisense compound.
4. The method of item 1 or 2, wherein the method further comprises administering to the human subject by intrathecal injection maintenance doses of the antisense compound or the salt thereof, beginning about 4 months after administration of the second loading dose and continuing once about every 4 months thereafter, wherein each maintenance dose is equivalent to about 28 mg of the antisense compound.
5. The method of item 4, wherein the second loading dose is administered about 14 days after administration of the first loading dose.
6. The method of item 1 or 2, wherein the method further comprises administering to the human subject by intrathecal injection:
   (iii) a third loading dose of the antisense compound or the salt thereof that is equivalent to about 50 mg of the antisense compound, administered about 10 to 20 days after administration of the second loading dose; and
   (iv) maintenance doses of the antisense compound or the salt thereof, beginning about 4 months after administration of the third loading dose and continuing once about every 4 months thereafter, wherein each maintenance dose is equivalent to about 28 mg of the antisense compound.
7. The method of item 6, wherein the second loading dose is administered about 14 days after administration of the first loading dose and the third loading dose is administered about 14 days after administration of the second loading dose.
8. The method of item 1 or 2, wherein the method further comprises administering to the human subject by intrathecal injection:
   (iii) a third loading dose of the antisense compound or the salt thereof that is equivalent to about 50 mg of the antisense compound, administered about 10 to 20 days after administration of the second loading dose;
   (iv) a fourth loading dose of the antisense compound or the salt thereof that is equivalent to about 50 mg of the antisense compound, administered about 10 to 20 days after administration of the third loading dose; and
   (v) maintenance doses of the antisense compound or the salt thereof, beginning about 4 months after administration of the fourth loading dose and continuing once about every 4 months thereafter, wherein each maintenance dose is equivalent to about 28 mg of the antisense compound.
9. The method of item 8, wherein the second loading dose is administered about 14 days after administration of the first loading dose, the third loading dose is administered about 14 days after administration of the second loading dose, and the fourth loading dose is administered about 14 days after administration of the third loading dose.
10. A method for treating spinal muscular atrophy (SMA) in a human subject in need thereof, the method comprising administering to the human subject an antisense compound having the following structure: 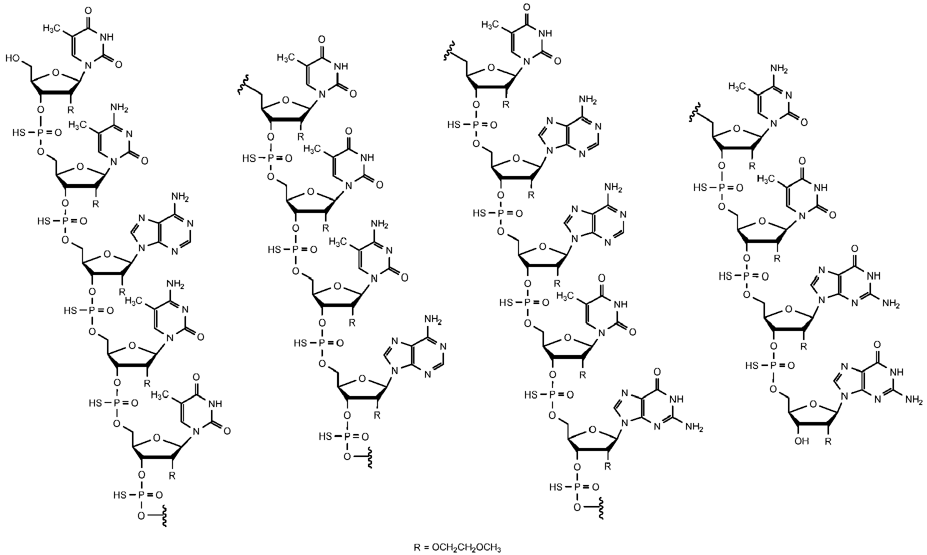 or a salt thereof,
   wherein the human subject was previously administered one or more doses of the antisense compound or the salt thereof, wherein the one or more previously administered doses were equivalent to about 12 mg of the antisense compound, wherein the method comprises administering to the human subject by intrathecal injection:
   (i) a loading dose of the antisense compound or the salt thereof that is equivalent to about 50 mg of the antisense compound, administered about 4 months after administration of the last previously administered dose equivalent to about 12 mg of the antisense compound; and
   (ii) maintenance doses of the antisense compound or the salt thereof, beginning about 4 months after administration of the loading dose and continuing once about every 4 months thereafter, wherein each maintenance dose is equivalent to about 28 mg of the antisense compound.
11. A method for increasing levels of survival motor neuron-2 (SMN2) messenger ribonucleic acid (mRNA) containing exon 7 in a human subject having mutations in chromosome 5q that are associated with SMN protein deficiency, the method comprising administering to the human subject an antisense compound having the following structure: 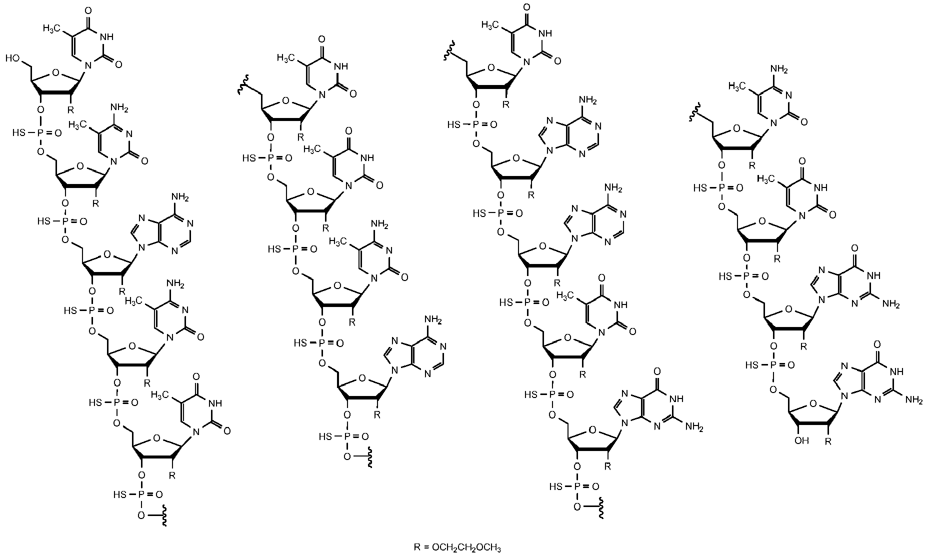 or a salt thereof,
   wherein the human subject was previously administered one or more doses of the antisense compound or the salt thereof, wherein the one or more previously administered doses were equivalent to about 12 mg of the antisense compound, wherein the method comprises administering to the human subject by intrathecal injection:
   (i) a loading dose of the antisense compound or the salt thereof that is equivalent to about 50 mg of the antisense compound, administered about 4 months after administration of the last previously administered dose equivalent to about 12 mg of the antisense compound; and
   (ii) maintenance doses of the antisense compound or the salt thereof, beginning about 4 months after administration of the loading dose and continuing once about every 4 months thereafter, wherein each maintenance dose is equivalent to about 28 mg of the antisense compound.
12. A method for treating spinal muscular atrophy (SMA) in a human subject in need thereof, the method comprising administering to the human subject an antisense compound having the following structure: 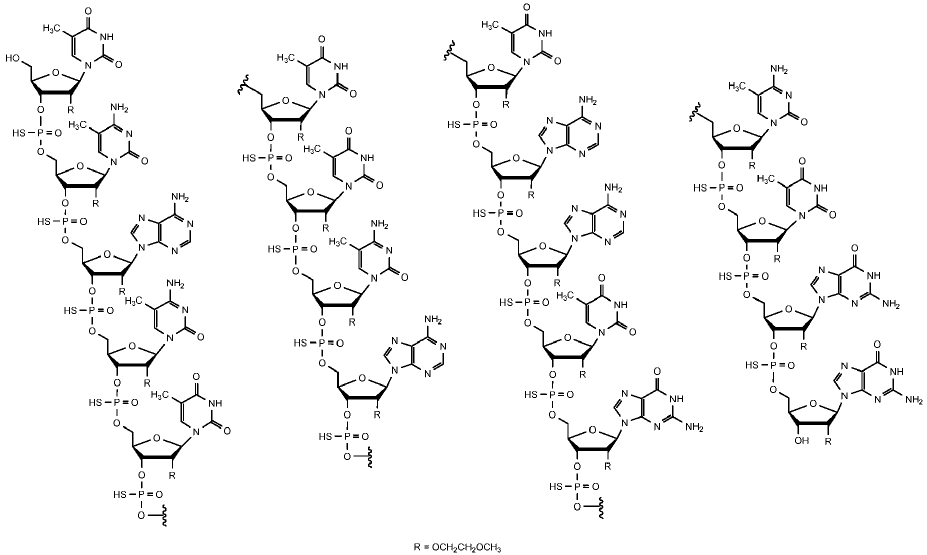 or a salt thereof,
   wherein the human subject was previously administered one or more doses of the antisense compound or the salt thereof, wherein the one or more previously administered doses were equivalent to about 12 mg of the antisense compound, wherein the method comprises administering to the human subject by intrathecal injection:
   (i) a first loading dose of the antisense compound or the salt thereof that is equivalent to about 28 mg of the antisense compound, administered about 4 months after administration of the last previously administered dose equivalent to about 12 mg of the antisense compound;
   (ii) a second loading dose of the antisense compound or the salt thereof that is equivalent to about 50 mg of the antisense compound, administered about 4 months after administration of the first loading dose; and
   (iii) maintenance doses of the antisense compound or the salt thereof, beginning about 4 months after administration of the second loading dose and continuing once about every 4 months thereafter, wherein each maintenance dose is equivalent to about 28 mg of the antisense compound.
13. A method for increasing levels of survival motor neuron-2 (SMN2) messenger ribonucleic acid (mRNA) containing exon 7 in a human subject having mutations in chromosome 5q that are associated with SMN protein deficiency, the method comprising administering to the human subject an antisense compound having the following structure: 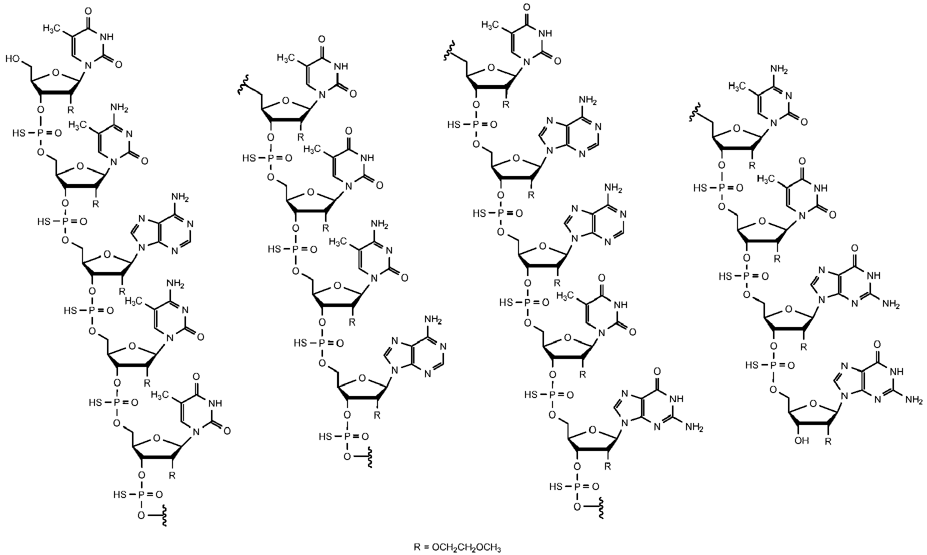 or a salt thereof,
   wherein the human subject was previously administered one or more doses of the antisense compound or the salt thereof, wherein the one or more previously administered doses were equivalent to about 12 mg of the antisense compound, wherein the method comprises administering to the human subject by intrathecal injection:
   (i) a first loading dose of the antisense compound or the salt thereof that is equivalent to about 28 mg of the antisense compound, administered about 4 months after administration of the last previously administered dose equivalent to about 12 mg of the antisense compound;
   (ii) a second loading dose of the antisense compound or the salt thereof that is equivalent to about 50 mg of the antisense compound, administered about 4 months after administration of the first loading dose; and
   (iii) maintenance doses of the antisense compound or the salt thereof, beginning about 4 months after administration of the second loading dose and continuing once about every 4 months thereafter, wherein each maintenance dose is equivalent to about 28 mg of the antisense compound.
14. The method of any one of items 10 to 13, wherein the human subject was previously administered at least five doses of the antisense compound or the salt thereof, wherein each of the at least five previously administered doses was equivalent to about 12 mg of the antisense compound.
15. The method of any one of items 1 to 14, wherein the human subject has (i) type I SMA; (ii) type II SMA; (iii) type III SMA; or (iv) type IV SMA.
16. The method of any one of items 1 to 14, wherein the human subject is presymptomatic for SMA.
17. The method of any one of items 1 to 16, wherein the human subject is administered the antisense compound or the salt thereof using a spinal anesthesia needle.
18. The method of any one of items 1 to 17, wherein the antisense compound or the salt thereof is administered in an injection volume of 5.0 mL
19. The method of item 18, wherein the antisense compound or the salt thereof is dissolved in phosphate buffered saline (PBS).
20. The method of item 18, wherein the antisense compound or the salt thereof is dissolved in artificial cerebrospinal fluid (aCSF).
21. The method of any one of items 1 to 20, wherein the salt of the antisense compound is a sodium salt.
22. The method of any one of items 1 to 20, wherein the salt of the antisense compound has the following structure: 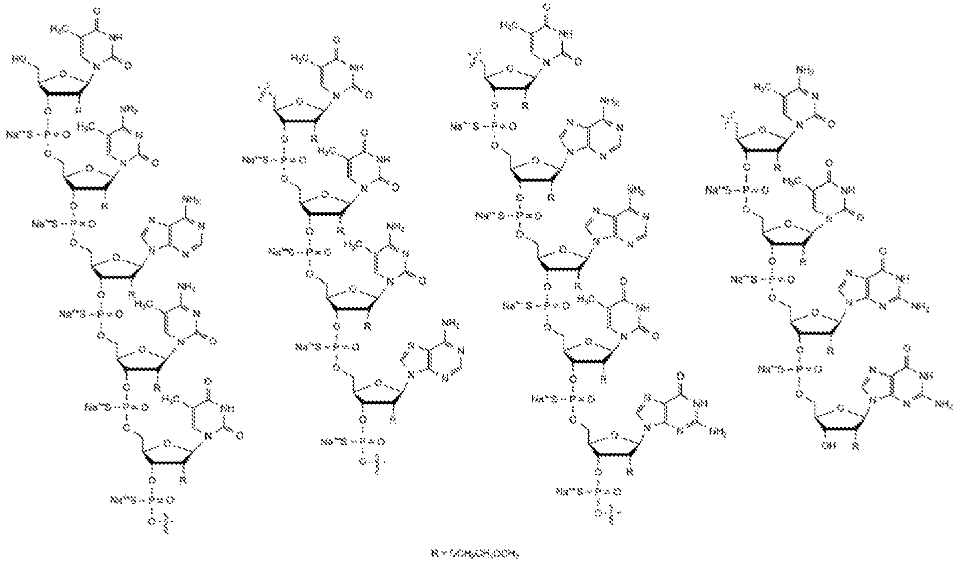
23. The method of any one of items 1 to 22, wherein the method comprises detecting a neurofilament level in the human subject before and/or after initiation of treatment.
24. The method of item 23, comprising:
   measuring a first neurofilament level in a first biological sample obtained from the human subject before initiation of treatment;
   administering the antisense compound or salt thereof to the human subject according to any one of the preceding claims; and
   measuring at least one subsequent neurofilament level in a second biological sample obtained from the human subject after administering all loading doses and at least one maintenance dose of the antisense compound or salt thereof.
25. The method of item 23, comprising:
   measuring a first neurofilament level in a first biological sample obtained from the human subject before initiation of treatment;
   administering a first loading dose of the antisense compound or salt thereof to the human subject according to any one of the preceding items; and
   measuring at least one subsequent neurofilament level in a biological sample obtained from the human subject after administering said first loading dose of the antisense compound or salt thereof.
26. The method of item 23, comprising:
   measuring a first neurofilament level in a first biological sample obtained from the human subject before initiation of treatment;
   administering a first loading dose and a second loading dose of the antisense compound or salt thereof to the human subject according to any one of the preceding claims; and
   measuring at least one subsequent neurofilament level in a biological sample obtained from the human subject after administering said first loading dose and said second loading dose of the antisense compound or salt thereof.
27. The method of any one of items 24-26, wherein the subsequent neurofilament level is reduced by at least 50% compared to the first neurofilament level.
28. The method of any one of items 25-26, wherein the subsequent neurofilament level is equal to or higher than the first neurofilament level and the method further comprises administering further doses of the antisense compound or salt thereof to the human subject.
29. The method of any one of items 23 to 28, wherein the neurofilament is a neurofilament heavy chain (NF-H), medium/intermediate chain (NF-M), or light chain (NF-L).
30. The method of items 23 to 29, wherein the first and second biological samples are blood, serum, plasma, or cerebrospinal fluid.

## Claims

1. An antisense compound having the following structure: 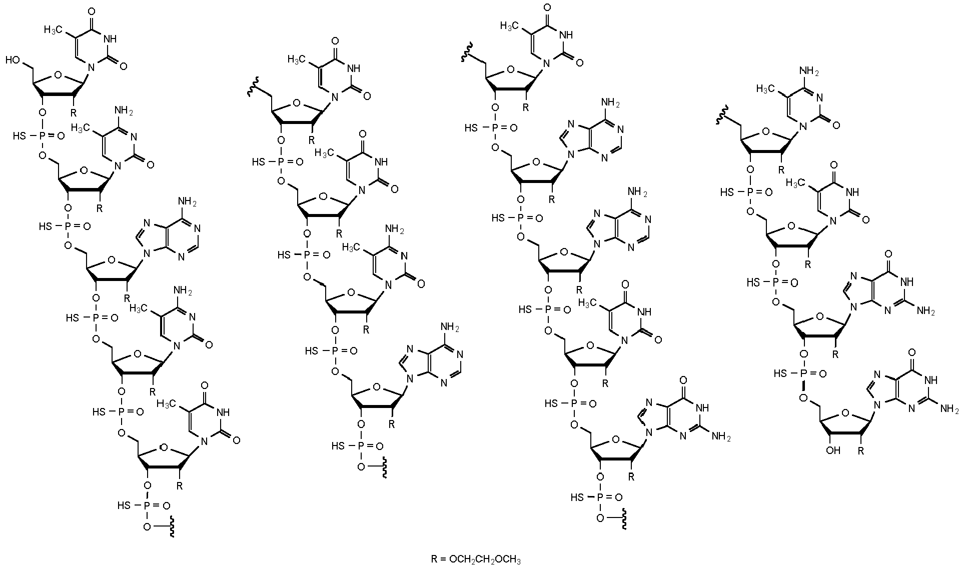 or a salt thereof for use in a method of increasing levels of survival motor neuron-2 (SMN2) messenger ribonucleic acid (mRNA) containing exon 7 in a human subject having mutations in chromosome 5q that are associated with SMN protein deficiency, the method comprising administering to the human subject at least two loading doses of the antisense compound or the salt thereof,
wherein the method comprises administering to the human subject intrathecally:
(i) a first loading dose of the antisense compound or the salt thereof that is equivalent to about 50 mg of the antisense compound; and
(ii) a second loading dose of the antisense compound or the salt thereof that is equivalent to about 50 mg of the antisense compound, administered about 10 to 20 days after administration of the first loading dose.

2. An antisense compound having the following structure: 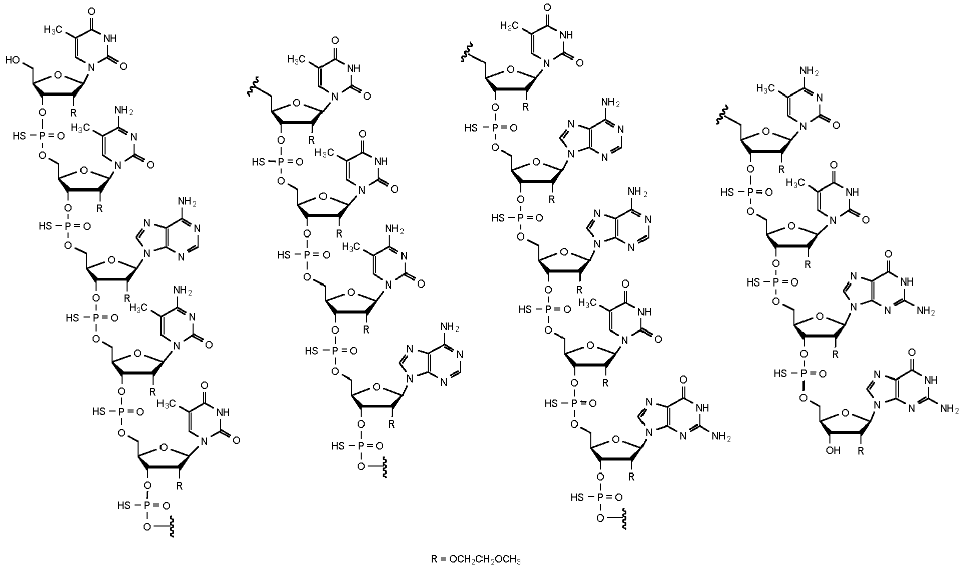 or a salt thereof for use in a method of increasing levels of survival motor neuron-2 (SMN2) messenger ribonucleic acid (mRNA) containing exon 7 in a human subject having mutations in chromosome 5q that are associated with SMN protein deficiency, the method comprising administering to the human subject the antisense compound or the salt thereof,
wherein the human subject was previously administered one or more doses of the antisense compound or the salt thereof, wherein the one or more previously administered doses were equivalent to about 12 mg of the antisense compound, wherein the method comprises administering to the human subject intrathecally:
(i) a loading dose of the antisense compound or the salt thereof that is equivalent to about 50 mg of the antisense compound, administered about 4 months after administration of the last previously administered dose equivalent to about 12 mg of the antisense compound; and
(ii) maintenance doses of the antisense compound or the salt thereof, beginning about 4 months after administration of the loading dose and continuing once about every 4 months thereafter, wherein each maintenance dose is equivalent to about 28 mg of the antisense compound.

3. An antisense compound having the following structure: 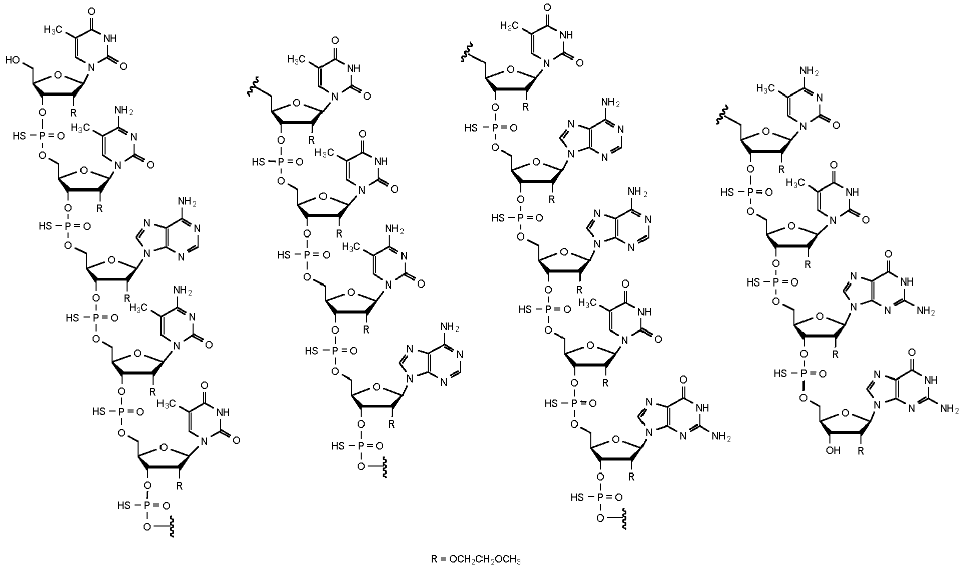 or a salt thereof for use in a method of treating spinal muscular atrophy (SMA) in a human subject in need thereof, the method comprising administering to the human subject the antisense compound or the salt thereof,
wherein the human subject was previously administered one or more doses of the antisense compound or the salt thereof, wherein the one or more previously administered doses were equivalent to about 12 mg of the antisense compound, wherein the method comprises administering to the human subject intrathecally:
(i) a first loading dose of the antisense compound or the salt thereof that is equivalent to about 28 mg of the antisense compound, administered about 4 months after administration of the last previously administered dose equivalent to about 12 mg of the antisense compound;
(ii) a second loading dose of the antisense compound or the salt thereof that is equivalent to about 50 mg of the antisense compound, administered about 4 months after administration of the first loading dose; and
(iii) maintenance doses of the antisense compound or the salt thereof, beginning about 4 months after administration of the second loading dose and continuing once about every 4 months thereafter, wherein each maintenance dose is equivalent to about 28 mg of the antisense compound.

4. An antisense compound having the following structure: 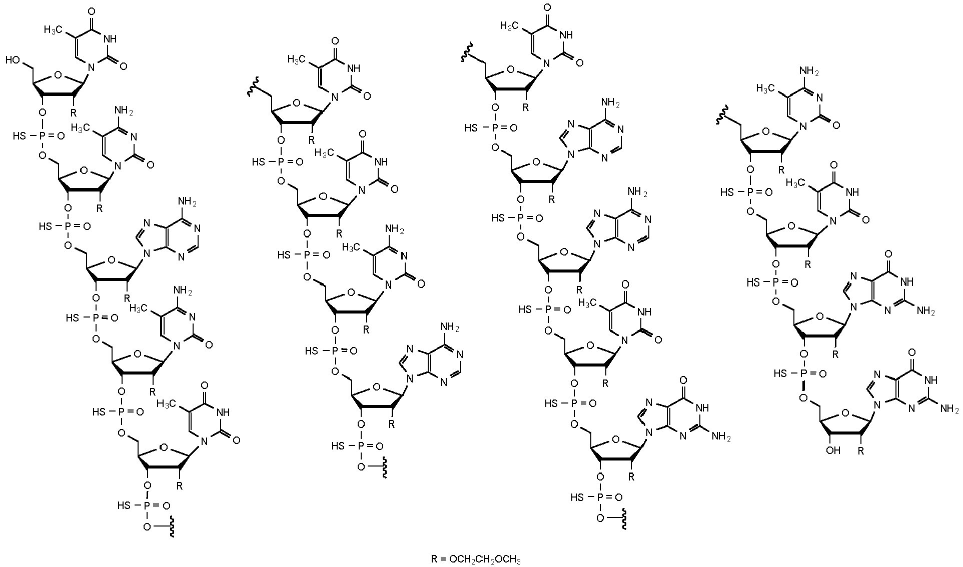 or a salt thereof for use in a method of increasing levels of survival motor neuron-2 (SMN2) messenger ribonucleic acid (mRNA) containing exon 7 in a human subject having mutations in chromosome 5q that are associated with SMN protein deficiency, the method comprising administering to the human subject an antisense compound or the salt thereof,
wherein the human subject was previously administered one or more doses of the antisense compound or the salt thereof, wherein the one or more previously administered doses were equivalent to about 12 mg of the antisense compound, wherein the method comprises administering to the human subject intrathecally:
(i) a first loading dose of the antisense compound or the salt thereof that is equivalent to about 28 mg of the antisense compound, administered about 4 months after administration of the last previously administered dose equivalent to about 12 mg of the antisense compound;
(ii) a second loading dose of the antisense compound or the salt thereof that is equivalent to about 50 mg of the antisense compound, administered about 4 months after administration of the first loading dose; and
(iii) maintenance doses of the antisense compound or the salt thereof, beginning about 4 months after administration of the second loading dose and continuing once about every 4 months thereafter, wherein each maintenance dose is equivalent to about 28 mg of the antisense compound.

5. The antisense compound for use in a method according to any one of claims 1 to 4, wherein the salt of the antisense compound has the following structure:

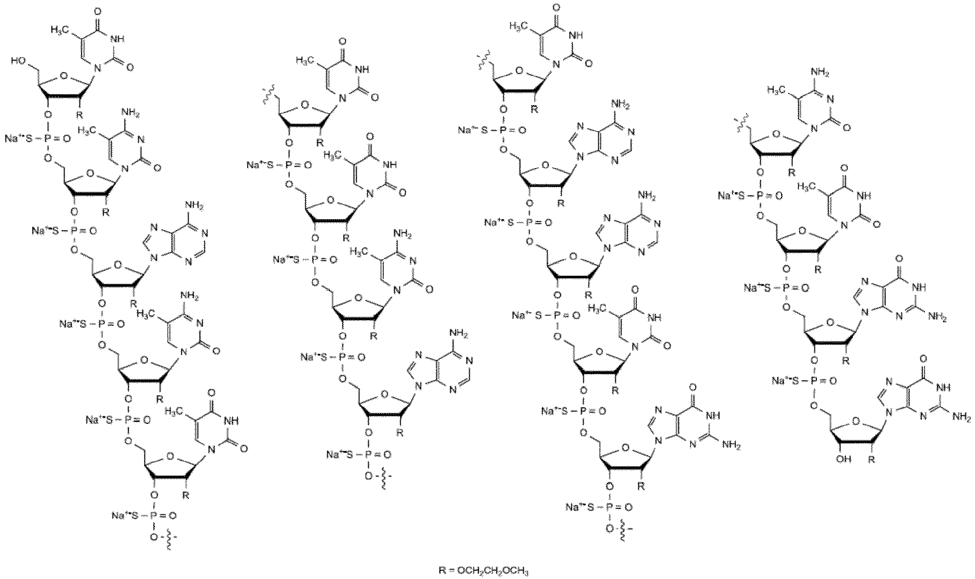

6. A kit comprising a pharmaceutical composition comprising an antisense compound having the following structure: 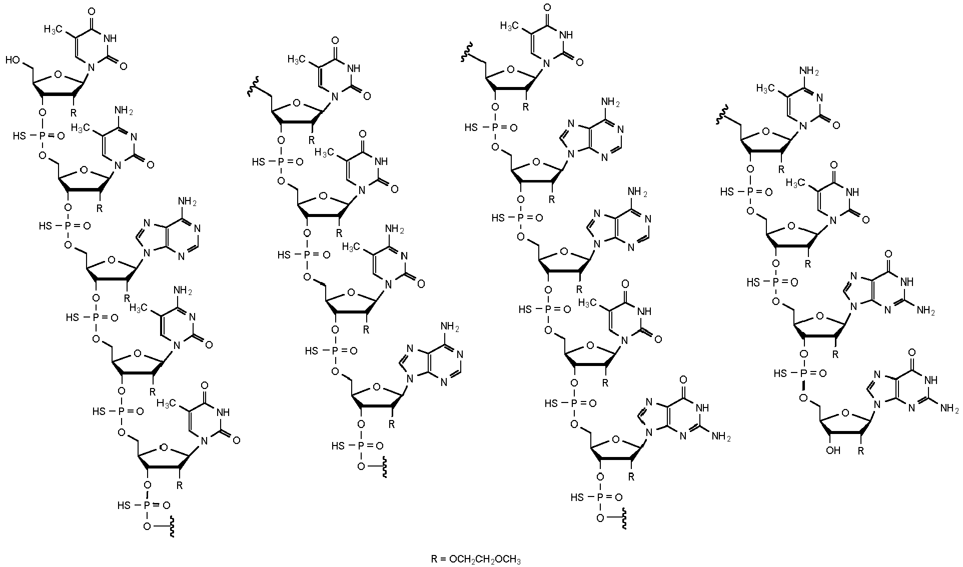 or a salt thereof, wherein the pharmaceutical composition comprises about 28 mg of the antisense compound or salt thereof.

7. A kit comprising a pharmaceutical composition comprising an antisense compound having the following structure: 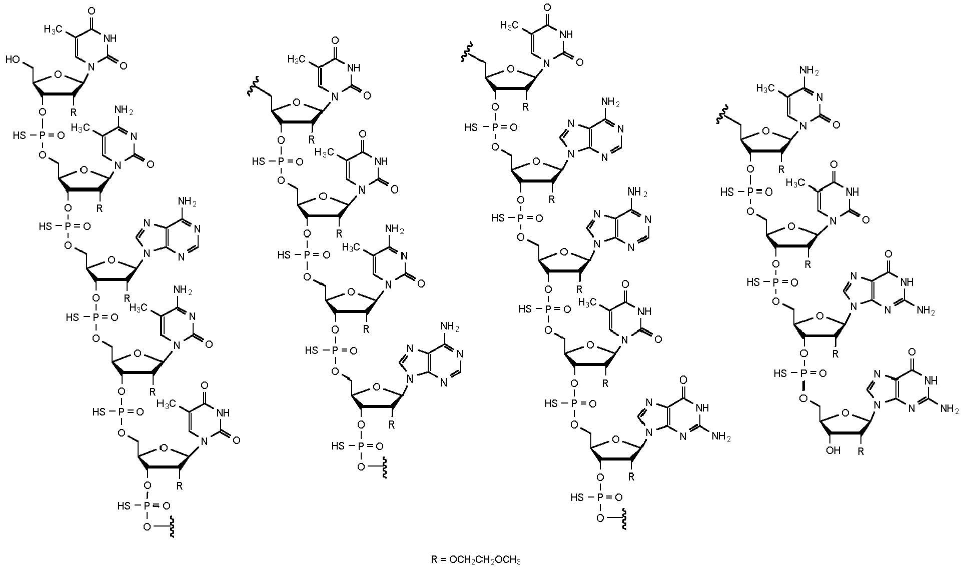 or a salt thereof, wherein the pharmaceutical composition comprises about 50 mg of the antisense compound or salt thereof.

8. A kit comprising a first pharmaceutical composition and a second pharmaceutical composition each of which comprises an antisense compound having the following structure: 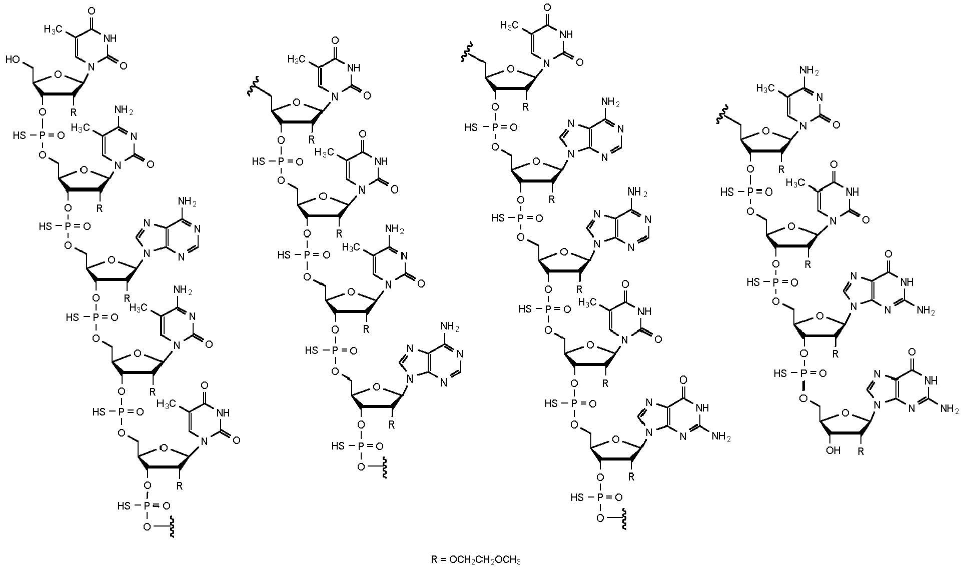 or a salt thereof, wherein the first pharmaceutical composition comprises about 28 mg of the antisense compound or salt thereof, and wherein the second pharmaceutical composition comprises about 50 mg of the antisense compound or salt thereof.

9. The kit of any one of claims 6 to 8, wherein the pharmaceutical composition comprises phosphate buffered saline (PBS) or artificial cerebrospinal fluid (aCSF).

10. The kit of any one of claims 6 to 9, wherein the antisense compound or salt thereof is at a concentration of 10 mg/mL or 5.6 mg/mL.

11. The kit of any one of claims 6 to 10, wherein the pharmaceutical composition is formulated for intrathecal administration, optionally for intrathecal bolus injection.

12. The kit of any one of claims 6 to 11, wherein the pharmaceutical composition comprises a salt of the antisense compound, optionally, the salt is sodium salt.

13. The kit of any one of claims 6 to 12, further comprising a syringe for injection and/or instructions for use.
